(19) Europäisches Patentamt
European Patent Office
Office européen des brevets

(11) EP 1 915 182 B1

(12) EUROPÄISCHE PATENTSCHRIFT

(45) Veröffentlichungstag und Bekanntmachung des Hinweises auf die Patenterteilung:
**08.07.2009 Patentblatt 2009/28**

(21) Anmeldenummer: **06724499.6**

(22) Anmeldetag: **21.04.2006**

(51) Int Cl.:
***A61L 15/60*** *(2006.01)*

(86) Internationale Anmeldenummer:
**PCT/EP2006/003695**

(87) Internationale Veröffentlichungsnummer:
**WO 2006/111403 (26.10.2006 Gazette 2006/43)**

# (54) MIT POLYKATIONEN OBERFLÄCHENBEHANDELTES WASSERABSORBIERENDE POLYMERGEBILDE

WATER-ABSORBENT POLYMER ENTITY, THE SURFACE THEREOF BEING TREATED BY POLYCATIONS

STRUCTURES POLYMERES ABSORBANT L'EAU TRAITEES EN SURFACE PAR POLYCATIONS

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **22.04.2005 DE 102005018922**

(43) Veröffentlichungstag der Anmeldung:
**30.04.2008 Patentblatt 2008/18**

(73) Patentinhaber: **Evonik Stockhausen GmbH**
**47805 Krefeld (DE)**

(72) Erfinder:
- **FURNO, Franck**
  **40545 Düsseldorf (DE)**
- **WALDEN, Mirko**
  **45699 Herten (DE)**
- **ISSBERNER, Jörg**
  **47877 Willich-neersen (DE)**
- **SCHMIDT, Harald**
  **47918 Tönisvorst (DE)**

(74) Vertreter: **Herzog, Martin**
**KNH Patentanwälte**
**Kahlhöfer Neumann Herzog Fiesser**
**Karlstrasse 76**
**40210 Düsseldorf (DE)**

(56) Entgegenhaltungen:
**EP-A- 0 412 363**
**WO-A-95/22357**
**US-A1- 2003 125 684**

EP 1 915 182 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft ein wasserabsorbierendes Polymergebilde, ein Verfahren zur Behandlung der Oberfläche wasserabsorbierender Polymergebilde, die durch dieses Verfahren erhältlichen oberflächenbehandelten, wasserabsorbierenden Polymergebilde, einen Verbund beinhaltend ein wasserabsorbierendes Polymergebilde sowie ein Substrat, ein Verfahren zur Herstellung eines Verbundes, den durch dieses Verfahren erhältlichen Verbund, chemische Produkte wie Schäume, Formkörper und Fasern beinhaltend wasserabsorbierende Polymergebilde oder einen Verbund, die Verwendung wasserabsorbierender Polymergebilde oder eines Verbundes in chemischen Produkten sowie die Verwendung von Verbindungen umfassend ein Polykation mit bestimmter Struktur zur Behandlung der Oberfläche wasserabsorbierender Polymergebilde.

Superabsorber sind wasserunlösliche, vernetzte Polymere, die in der Lage sind, unter Quellung und Ausbildung von Hydrogelen große Mengen an wässrigen Flüssigkeiten, insbesondere Körperflüssigkeiten, vorzugsweise Urin oder Blut, aufzunehmen und unter einem bestimmten Druck zurückzuhalten. Durch diese charakteristischen Eigenschaften finden diese Polymere hauptsächlich Anwendung bei der Einarbeitung in Sanitärartikeln, wie beispielsweise Babywindeln, Inkontinenzprodukten oder Damenbinden.

Die Herstellung der Superabsorber erfolgt in der Regel durch die radikalische Polymerisation säuregruppentragender Monomere in Gegenwart von Vemetzem. Dabei lassen sich durch die Auswahl der Monomerenzusammensetzung, der Vernetzer sowie der Polymerisationsbedingungen und der Verarbeitungsbedingungen für das nach der Polymerisation erhaltene Hydrogel Polymere mit unterschiedlichen Absorbereigenschaften herstellen. Weitere Möglichkeiten bietet die Herstellung von Pfropfpolymerisaten, beispielsweise unter Verwendung chemisch modifizierter Stärke, Cellulose und Polyvinylalkohol nach DE-OS 26 12 846 und die Nachbehandlung der Hydrogele oder der nach dem Trocknen der Hydrogele erhaltnen pulverförmigen Polymerteilchen durch Nachvernetzung der Oberflächen der Polymerteilchen, beispielsweise nach DE 40 20 780 C1. Durch die Nachvernetzung der Oberfläche der wasserabsorbierenden Polymerteilchen wird insbesondere das Absorptionsvermögen der Polymerteilchen unter der Einwirkung von Drücken erhöht.

EP 0 248 963 A2 schlägt vor, zur Verbesserung der Aufnahmekapazität und der Retention von Wasser die wasserabsorbierenden Polymerteilchen mit polyquarternären Aminen zu beschichten. Der Nachteil der in EP 0 248 973 A2 beschriebenen wasserabsorbierenden Polymerteilchen besteht jedoch darin, dass diese zwar in Folge der Beschichtung mit den dort eingesetzten quartemären Aminen eine verbesserte Aufnahmekapazität und eine verbesserte Retention zeigen, absorbierende Strukturen, welche diese Polymere in hohen Konzentrationen beinhalten, jedoch durch ein unbefriedigendes Absorptionsverhalten gekennzeichnet sind. Diese unbefriedigende Absorptionsverhalten manifestiert sich insbesondere durch den sogenannten "Gel-Blocking"-Effekt. Unter diesem "Gel-Blocking"-Effekt wird die Beobachtung verstanden, dass in absorbierenden Strukturen die Diffusion von Flüssigkeiten in die Struktur hinein aufgrund der beschränkten Permeabilität der absorbierenden Struktur geringer ist als die Geschwindigkeit, mit der die Flüssigkeit in die absorbierender Struktur eintritt, so dass es, wenn es sich beispielsweise bei der absorbierenden Struktur um eine Windel handelt, zu einem Flüssigkeitsaustritt kommen kann.

Der vorliegenden Erfindung lag daher die Aufgabe zugrunde, die sich aus dem Stand der Technik ergebenden Nachteile zu überwinden.

Insbesondere lag der vorliegenden Erfindung die Aufgabe zugrunde, Superabsorber sowie absorbierende Strukturen beinhaltend diese Superabsorber in hohen Mengen bereitzustellen, die neben ausgezeichneten Absorptions- und Retentionseigenschaften auch dadurch besonders vorteilhafte Permeabilitätseigenschaften gekennzeichnet sind.

Weiterhin lag der vorliegenden Erfindung die Aufgabe zugrunde, Verfahren anzugeben, mit dem Superabsorber sowie einen Verbund beinhaltend diese Superabsorber mit den vorstehend genannten Vorteilen hergestellt werden können.

Einen Beitrag zur Lösung der vorstehend genannten Aufgaben liefert ein wasserabsorbierendes Polymergebilde, dessen Oberfläche mit einer Verbindung umfassend ein Polykation und mindestens ein Anion in Kontakt gebracht worden ist und welches vorzugsweise eine gemäß ERT 442.2-02 (ERT = Edana Recommended Test Method) bestimmte Absorption unter einem Druck von 50 g/cm$^2$ von mindestens 16 g/g, besonders bevorzugt von mindestens 18 g/g und am meisten bevorzugt von mindestens 20 g/g aufweist, wobei vorzugsweise eine Absorption unter einem Druck von 50 g/cm$^2$ von 100 g/g, besonders bevorzugt von 50 g/g und darüber hinaus bevorzugt von 40 g/g nicht überschritten wird (im Falle von Partikeln jeweils bestimmt für die gesamte Partikelfraktion).

Einen Beitrag zur Lösung der vorstehend genannten Aufgaben liefert auch ein wasserabsorbierendes Polymergebilde, dessen Oberfläche mit einer Verbindung umfassend ein Polykation und mindestens ein Anion in Kontakt gebracht worden ist, wobei die Verbindung umfassend ein Polykation und mindestens ein Anion ein Gewichtsmittel des Molekulargewichtes von mehr als 3.000 g/mol, vorzugsweise mehr als 4.000 g/mol, noch mehr bevorzugt mehr als 5.000 g/mol, darüber hinaus noch mehr bevorzugt mehr als 7.000 g/mol und am meisten bevorzugt mehr als 10.000 g/mol aufweist, wobei vorzugsweise ein Gewichtsmittel des Molekulargewichtes von 100.000.000 g/mol, besonders bevorzugt von 10.000.000 g/mol nicht überschritten wird.

In einer bevorzugten Ausfiiluungsform der erfindungsgemäßen wasserabsorbierenden Polymergebilde sind diese oberflächennachvemetzt. Oberflächennachvernetzte Polymergebilde sind dadurch gekennzeichnet, dass sie einen Innenbereich und einen den Innenbereich umgebenden Aussenbereich aufweisen, wobei der Aussenbereich einen höheren Vernetzungsgrad aufweist als der Innenbereich.

Erfindungsgemäß bevorzugte Polymergebilde sind Fasern, Schäume oder Teilchen, wobei Fasern und Teilchen bevorzugt und Teilchen besonders bevorzugt sind.

Erfindungsgemäß bevorzugte Polymerfasern sind so dimensioniert, dass sie in oder als Garne für Textilien und auch direkt in Textilien eingearbeitet werden können. Es ist erfindungsgemäß bevorzugt, dass die Polymerfasern eine Länge im Bereich von 1 bis 500 mm, bevorzugt 2 bis 500 mm und besonders bevorzugt 5 bis 100 mm und einen Durchmesser im Bereich von 1 bis 200 Denier, bevorzugt 3 bis 100 Denier und besonders bevorzugt 5 bis 60 Denier besitzen.

Erfindungsgemäß bevorzugte Polymerteilchen sind so dimensioniert, dass sie eine mittlere Teilchengröße gemäß ERT 420.2-02 im Bereich von 10 bis 3000 $\mu$m, vorzugsweise 20 bis 2000 $\mu$m und besonders bevorzugt 150 bis 850 $\mu$m oder 150 bis 600 $\mu$m aufweisen. Dabei ist es insbesondere bevorzugt, dass der Anteil der Polymerteilchen mit einer Partikelgröße in einem Bereich von 300 bis 600 $\mu$m mindestens 30 Gew.%, besonders bevorzugt mindestens 40 Gew.-%, darüber hinaus bevorzugt mindestens 50 Gew.% und am meisten bevorzugt mindestens 75 Gew.-%, bezogen auf das Gesamtgewicht der nachvernetzten, wasserabsorbierenden Polymerteilchen, beträgt. Gemäß einer anderen Ausführungsform der erfindungsgemäßen wasserabsorbierenden Polymergebilde beträgt der Anteil der Polymerteilchen mit einer Partikelgröße in einem Bereich von 150 bis 850 $\mu$m mindestens 50 Gew.-%, besonders bevorzugt mindestens 75 Gew.-%, darüber hinaus bevorzugt mindestens 90 Gew.-% und am meisten bevorzugt mindestens 95 Gew.-%, bezogen auf das Gesamtgewicht der nachvernetzten, wasserabsorbierenden Polymerteilchen.

In einer bevorzugten Ausfiihrungsform der erfindungsgemäßen wasserabsorbierenden Polymergebilde basieren diese auf

($\alpha$1) 20-99,999 Gew.-%, bevorzugt 55-98,99 Gew.-% und besonders bevorzugt 70-98,79 Gew.-% polymerisierten, ethylenisch ungesättigten, säuregruppen-tragenden Monomeren oder deren Salze oder polymerisierten, ethylenisch ungesättigten, einen protonierten oder quarternierten Stickstoff beinhaltenden Monomeren, oder deren Mischungen, wobei mindestens ethylenisch ungesättigte, säuregruppenhaltige Monomere, vorzugsweise Acrylsäure, beinhaltende Mischungen besonders bevorzugt sind,

($\alpha$2) 0-80 Gew.-%, vorzugsweise 0-44,99 Gew.-% und besonders bevorzugt 0,1-44,89 Gew.-% polymerisierten, monoethylenisch ungesättigten, mit ($\alpha$1) copolymerisierbaren Monomeren,

($\alpha$3) 0,001-5 Gew.-%, vorzugsweise 0,01-3 Gew.-% und besonders bevorzugt 0,01-2,5 Gew.-% eines oder mehrerer Vernetzer,

($\alpha$4) 0,001-10 Gew.-%, vorzugsweise 0,01-7,5 Gew.-% und am meisten bevorzugt 0,5-5 Gew.-% der Verbindung umfassend mindestens ein Anion und ein Polykation,

($\alpha$5) 0-30 Gew.-%, vorzugsweise 0-5 Gew.-% und besonders bevorzugt 0,1-5 Gew.-% eines wasserlöslichen Polymeren,

($\alpha$6) 0-20 Gew.-%, vorzugsweise 2,5-15 Gew.-% und besonders bevorzugt 5-10 Gew.-% Wasser, sowie

($\alpha$7) 0-20 Gew.-%, vorzugsweise 0-10 Gew.-% und besonders bevorzugt 0,1-8 Gew.-% eines oder mehrerer Hilfsmittel, wobei die Summe der Gewichtsinengen ($\alpha$1) bis ($\alpha$7) 100 Gew.-% beträgt.

Die monoethylenisch ungesättigten, säuregruppenhaltigen Monomere ($\alpha$1) können teilweise oder vollständig, bevorzugt teilweise neutralisiert sein. Vorzugsweise sind die monoethylenisch ungesättigten, säuregruppenhaltigen Monomere zu mindestens 25 Mol-%, besonders bevorzugt zu mindestens 50 Mol-% und darüber hinaus bevorzugt zu 50-80 Mol-% neutralisiert. In diesem Zusammenhang wird auf DE 195 29 348 A1 verwiesen, deren Offenbarung hiermit als Referenz eingeführt wird. Die Neutralisation kann teilweise oder ganz auch nach der Polymerisation erfolgen. Ferner kann die Neutralisation mit Alkalimetallhydroxiden, Erdalkalimetallhydroxiden, Ammoniak sowie Carbonaten und Bicarbonaten erfolgen. Daneben ist jede weitere Base denkbar, die mit der Säure ein wasserlösliches Salz bildet. Auch eine Mischrieutralisation mit verschiedenen Basen ist denkbar. Bevorzugt ist die Neutralisation mit Ammoniak und Alkalimetallhydroxiden, besonders bevorzugt mit Natriumhydroxid und mit Ammoniak.

Ferner können bei einem Polymer die freien Säuregruppen überwiegen, so dass dieses Polymer einen im sauren Bereich liegenden pH-Wert aufweist. Dieses saure wasserabsorbierende Polymer kann durch ein Polymer mit freien basischen Gruppen, vorzugsweise Amingruppen, das im Vergleich zu dem sauren Polymer basisch ist, mindestens teilweise neutralisiert werden. Diese Polymere werden in der Literatur als "Mixed-Bed Ion-Exchange Absorbent Polymers" (MBIEA-Polymere) bezeichnet und sind unter anderem in der WO 99/34843 A1 offenbart. Die Offenbarung der WO 99/34843 A1 wird hiermit als Referenz eingeführt und gilt somit als Teil der Offenbarung. In der Regel stellen MBIEA-Polymere eine Zusammensetzung dar, die zum einen basische Polymere, die in der Lage sind, Anionen auszutauschen, und andererseits ein im Vergleich zu dem basischen Polymer saures Polymer, das in der Lage ist, Kationen auszutauschen, beinhalten. Das basische Polymer weist basische Gruppen auf und wird typischerweise durch die Polymerisation von Monomeren erhalten, die basische Gruppen oder Gruppen tragen, die in basische Gruppen umgewandelt werden können. Bei diesen Monomeren handelt es sich vor allen Dingen um solche, die primäre, sekundäre oder tertiäre Amine oder die entsprechenden Phosphine oder mindestens zwei der vorstehenden funktionellen Gruppen aufweisen. Zu dieser Gruppe von Monomeren gehören insbesondere Ethylenamin, Allylamin, Diallylamin, 4-Aminobuten, Alkyloxycycline, Vinylformamid, 5-Aminopenten, Carbodiimid, Formaldacin, Melamin und dergleichen, sowie deren sekundäre oder tertiäre Aminderivate.

Bevorzugte ethylenisch ungesättigte, säuregruppenhaltige Monomere ($\alpha$1) sind vorzugsweise diejenigen Verbindungen, die in der WO 2004/037903 A2, die hiermit als Referenz eingeführt wird und somit als Teil der Offenbarung gilt, als ethylenisch ungesättigte, säuregruppenhaltige Monomere ($\alpha$1) genannt werden. Besonders bevorzugte ethylenisch ungesättigte, säuregruppenhaltige Monomere ($\alpha$1) sind Acrylsäure und Methacrylsäure, wobei Acrylsäure am meisten bevorzugt ist.

Gemäß einer Ausführungsform des erfindungsgemäßen Verfahrens werden wasserabsorbierende Polymergebilde eingesetzt, bei denen die monoethylenisch ungesättigten, mit ($\alpha$1) copolymerisierbare Monomere ($\alpha$2) Acrylamide, Methacrylamide oder Vinylamide sind.

Bevorzugte (Meth)acrylamide sind neben Acrylamid und Methacrylamid alkylsubstituierte (Meth)acrylamide oder aminoalkylsubstituierte Derivate des (Meth)acrylamids, wie N-Methylol(meth)acrylamid, N,N-Dimethylamino-(meth) acrylamid, Dimethyl(meth)acrylamid oder Diethyl(meth)acrylamid. Mögliche Vinylamide sind beispielsweise N-Vinylamide, N-Vinylformamide, N-Vinylacetamide, N-Vinyl-N-Methylacetamide, N-Vinyl-N-methylformamide, Vinylpyrrolidon. Unter diesen Monomeren besonders bevorzugt ist Acrylamid.

Gemäß einer anderen Ausführungsform des erfindungsgemäßen Verfahrens werden wasserabsorbierende Polymergebilde eingesetzt, bei denen die monoethylenisch ungesättigten, mit ($\alpha$1) copolymerisierbare Monomere ($\alpha$2) wasserlösliche Monomere sind. In diesem Zusammenhang sind insbesondere Alkoxypolyalkylenoxid(meth)acrylate wie Methoxypolyethylenglykol(meth)acrylate bevorzugt.

Des Weiteren sind als monoethylenisch ungesättigte, mit ($\alpha$1) copolymerisierbaren Monomere ($\alpha$2) in Wasser dispergierbare Monomere bevorzugt. Als in Wasser dispergierbare Monomere sind Acrylsäureester und Methacrylsäureester, wie Methyl(meth)acrylat, Ethyl(meth)acrylat, Propyl(meth)acrylat oder Butyl(meth)-acrylat.

Die monoethylenisch ungesättigten, mit ($\alpha$1) copolymerisierbaren Monomere ($\alpha$2) umfassen weiterhin Methylpolyethylenglykol-allylether, Vinylacetat, Styrol und Isobutylen.

Als Vernetzer ($\alpha$3) werden vorzugsweise diejenigen Verbindungen eingesetzt, die in der WO 2004/037903 A2 als Vernetzer ($\alpha$3) genannt werden. Unter diesen Vemetzem sind wasserlösliche Vernetzer besonders bevorzugt. Am meisten bevorzugt sind dabei N,N'-Methylenbisacrylamid; Polyethylenglykol-di(meth)acrylate, Triallylmethylammoniumchlorid, Tetraallylammoniumchlorid sowie mit 9 Mol Ethylenoxid pro Mol Acrylsäure hergestelltes Allylnonaethylenglykolacrylat besonders bevorzugt.

Als Verbindung ($\alpha$4) umfassend mindestens ein Anion und ein Polykation sind Verbindungen bevorzugt, in denen das Polykation die Struktur I

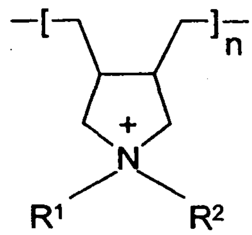

Struktur I

aufweist, in der

| | |
|---|---|
| $R^1$ und $R^2$ | gleich oder verschieden sein können und ein Kohlenwasserstoffrest mit 1 bis 10 Kohlenstoffatomen, vorzugsweise 1 bis 7 Kohlenstoffatomen, besonders bevorzugt 1 bis 4 Kohlenstoffatomen, darüber hinaus bevorzugt 1 bis 2 Kohlenstoffatomen und am meisten bevorzugt eine Methylgruppe darstellen und |
| n | einen Wert von mindestens 25, vorzugsweise mindestens 31, noch mehr bevorzugt mindestens 100, |

darüber hinaus bevorzugt mindestens 1.000, noch mehr bevorzugt mindestens 2.000, darüber hinaus noch mehr bevorzugt mindestens 2.500, noch mehr bevorzugt mindestens 3.000 und am meisten bevorzugt mindestens 5.000 hat.

Besonders bevorzugte Polykationen der Struktur I sind Polydiallyldimethylamine. Bevorzugte Anionen sind das Chlorid-Ion, das Bromid-Ion, das Iodid-Ion, das Sulfid-Ion, das Sulfat-Ion, das Sulfit-Ion, das Nitrat-Ion, das Nitrit-Ion, das Acetat-Ion, das Lactat-Ion, das Carbonat-Ion, das Hydrogencarbonat-Ion, das Phosphat-Ion, das Phosphit-Ion und das Hydroxid-Ion. Neben diesen mono- bzw. divalenten Anionen kann die Verbindung auch polyvalente Anionen umfassen. Eine besonders bevorzugte Verbindung beinhaltend mindestens ein Anion und ein Polykation ist ein Polymer aus Diallyldimethylammoniumchlorid mit einem Wert für n von mindestens 25, vorzugsweise mindestens 31, darüber hinaus bevorzugt mindestens 100, darüber hinaus noch mehr bevorzugt mindestens 1.000, weiter bevorzugt mindestens 2.500 und am meisten bevorzugt mindestens 5.000.

Als wasserlösliche Polymere ($\alpha$5) können in den Polymergebilden wasserlösliche Polymerisate, wie teil- oder vollverseifter Polyvinylalkohol, Polyvinylpyrrolidon, Stärke oder Stärkederivate, Polyglykole oder Polyacrylsäure enthalten, vorzugsweise einpolymerisiert sein. Das Molekulargewicht dieser Polymere ist unkritisch, solange sie wasserlöslich sind. Bevorzugte wasserlösliche Polymere sind Stärke oder Stärkederivate oder Polyvinylalkohol. Die wasserlöslichen Polymere, vorzugsweise synthetische wie Polyvinylalkohol, können auch als Pfropfgrundlage für die zu polymerisierenden Monomeren dienen.

Als Hilfsmittel ($\alpha$6) sind vorzugsweise Stellmittel, Geruchsbinder, oberflächenaktive Mittel oder Antioxidatien sowie diejenigen Additive, die zur Herstellung der Polymergebilde eingesetzt wurden (Initiatoren usw.) in den Polymergebilden enthalten.

In einer besonderen Ausführungsform der erfindungsgemäßen Polymergebilde basieren diese zu mindestens 50 Gew.-%, vorzugsweise zu mindestens 70 Gew.-% und darüber hinaus bevorzugt zu mindestens 90 Gew.-% auf carboxylatgruppentragenden Monomeren. Es ist erfindungsgemäß weiterhin bevorzugt, dass die Komponente ($\alpha$1) zu mindestens 50 Gew.-%, vorzugsweise zu mindestens 70 Gew.-% aus Acrylsäure besteht, die vorzugsweise zu mindestens 20 Mol-%, besonders bevorzugt zu mindestens 50 Mol-% und darüber hinaus bevorzugt in einem Bereich von 60 bis 85 Mol-% neutralisiert ist.

Weiterhin ist anzumerken, dass die Verbindung umfassend das mindestens eine Anion sowie das Polykation nicht im gesamten Oberflächenbereich immobilisiert sein muss. Es ist jedoch erfindungsgemäß bevorzugt, dass auf mindestens 5 %, vorzugsweise auf mindestens 10 %, noch mehr bevorzugt auf mindestens 20 % und am meisten bevorzugt auf mindestens 30 % der äußeren Oberfläche diese Verbindung immobilisiert ist.

Weiterhin sind die erfindungsgemäßen Polymergebilde vorzugsweise durch mindestens eine der nachfolgenden Eigenschaften gekennzeichnet:

($\beta$1) kein Verbacken nach drei Stunden, vorzugsweise nach 6 Stunden und besonders bevorzugt nach 12 Stunden in dem in der WO 00/10619 A1 beschriebenen "anti-caking"-Test;

($\beta$2) bei einem gemäß ERT 441.2-02 bestimmten CRC-Wert (CRC = Centrifugation Retention Capacity; im Falle von Partikeln jeweils bestimmt für die gesamte Partikelfraktion) von weniger als 26 g/g einen gemäß der hierin beschriebenen Testmethode bestimmten SFC-Wert (SFC = Saline Flow Conductivity) von mindestens 80 $\times$ $10^{-7}cm^3$ $s\ g^{-1}$, vorzugsweise von mindestens 90 $\times$ $10^{-7}$ $cm^3$ $s\ g^{-1}$ und am meisten bevorzugt von mindestens 100 $\times$ $10^{-7}$ $cm^3 s\ g^{-1}$;

($\beta$3) bei einem gemäß ERT 441.2-02 bestimmten CRC-Wert im Bereich von 26 bis < 27 g/g einen gemäß der hierin beschriebenen Testmethode bestimmten SFC-Wert von mindestens 70 $\times$ $10^{-7}cm^3$ $s\ g^{-1}$, vorzugsweise von mindestens 80 $\times$ $10^{-7}cm^3$ $s\ g^{-1}$ und am meisten bevorzugt von mindestens 90 $\times$ $10^{-7}cm^3$ $s\ g^{-1}$;

($\beta$4) bei einem gemäß ERT 441.2-02 bestimmten CRC-Wert im Bereich von 27 bis < 28 g/g einen gemäß der hierin beschriebenen Testmethode bestimmten SFC-Wert von mindestens 60 $\times$ $10^{-7}cm^3$ $s\ g^{-1}$, vorzugsweise von mindestens 70 $\times$ $10^{-7}cm^3 s\ g^{-1}$ und am meisten bevorzugt von mindestens 80 $\times$ $10^{-7}cm^3 s\ g^{-1}$;

($\beta$5) bei einem gemäß ERT 441.2-02 bestimmten CRC-Wert im Bereich von 28 bis < 29 g/g einen gemäß der hierin beschriebenen Testmethode bestimmten SFC-Wert von mindestens 45 $\times$ $10^{-7}cm^3$ $s\ g^{-1}$, vorzugsweise von mindestens 55 $\times$ $10^{-7}cm^3$ $s\ g^{-1}$ und am meisten bevorzugt von mindestens 65 $\times$ $10^{-7}cm^3 s\ g^{-1}$;

($\beta$6) bei einem gemäß ERT 441.2-02 bestimmten CRC-Wert im Bereich von 29 bis < 30 g/g einen gemäß der hierin beschriebenen Testmethode bestimmten SFC-Wert von mindestens 30 $\times$ $10^{-7}cm^3$ $s\ g^{-1}$, vorzugsweise von mindestens 40 $\times$ $10^{-7}cm^3 s\ g^{-1}$ und am meisten bevorzugt von mindestens 50 $\times$ $10^{-7}cm^3 s\ g^{-1}$;

($\beta$7) bei einem gemäß ERT 441.2-02 bestimmten CRC-Wert im Bereich von 30 bis < 31 g/g einen gemäß der hierin beschriebenen Testmethode bestimmten SFC-Wert von mindestens 20 $\times$ $10^{-7}cm^3$ $s\ g^{-1}$, vorzugsweise von mindestens 30 $\times$ $10^{-7}cm^3$ $s\ g^{-1}$ und am meisten bevorzugt von mindestens 40 $\times$ $10^{-7}cm^3$ $s\ g^{-1}$;

($\beta$8) bei einem gemäß ERT 441.2-02 bestimmten CRC-Wert von mindestens 31 g/g einen gemäß der hierin beschriebenen Testmethode bestimmten SFC-Wert von mindestens 10 $\times$ $10^{-7}cm^3 s\ g^{-1}$, vorzugsweise von mindestens

20 × $10^{-7}cm^3$ s $g^{-1}$ und am meisten bevorzugt von mindestens 30 × $10^{-7}cm^3$ s $g^{-1}$ ;
(β9) einen gemäß der hierin beschriebenen Testmethode bestimmten Wicking-Index nach einer Minute von mindestens 7,5 cm, vorzugsweise mindestens 8,5 cm und am meisten bevorzugt mindestens 10,0 cm;
(β10) einen Neutralisationsgrad von höchstens 75 Mol-%, vorzugsweise höchstens 70 Mol-% und am meisten bevorzugt höchstens 65 Mol-%.

Vorzugsweise wird ein SFC-Wert von 750 × $10^{-7}cm^3$ s $g^{-1}$ bevorzugt von 500 × $10^{-7}cm^3$ s $g^{-1}$ darüber hinaus bevorzugt von 300 × $10^{-7}cm^3$ s $g^{-1}$ noch mehr bevorzugt von 260 × $10^{-7}cm^3$ s $g^{-1}$ und am meisten bevorzugt von 249 × $10^{-7}cm^3$ s $g^{-1}$ nicht überschritten. Es ist ferner bevorzugt, dass das erfindungsgemäße Polymergebilde einen Wicking Index nach einer Minute von maximal 100 cm, bevorzugt von maximal 75 cm, besonders bevorzugt von maximal 50 cm, darüber hinaus bevorzugt von maximal 25 cm and am meisten bevorzugt von maximal 10,5 cm aufweist.

Weiterhin bevorzugte Ausfiihrungsformen der erfindungsgemäßen Polymergebilde weisen jede denkbare Kombination der vorstehenden Merkmales (β1) bis (β10) auf, wobei die Ausführungsformen der folgenden Merkmalskombinationen bevorzugt sind: (β1), (β2), (β3), (β4), (β5), (β6), (β7), (β8), (β9), (β10), (β1)(β9), (β2)(β3)(β4)(β5)(β6)(β7)(β8)(β9) und (β1)(β2)(β3)(β4)(β5)(β6)(β7)(β8)(β9)(β10), wobei (β9), (β1)(β9) und (β1)(β2)(β3)(β4)(β5)(β6)(β7)(β8)(β9)(β10) am meisten bevorzugte Eigenschaftskombinationen sind.

Einen Beitrag zur Lösung der eingangs genannten Aufgaben liefert auch ein Verfahren zur Behandlung der Oberfläche wasserabsorbierender Polymergebilde, umfassend die Verfahrensschritte:

i) Bereitstellen eines unbehandelten, wasserabsorbierenden Polymergebildes,
ii) in Kontakt bringen des unbehandelten wasserabsorbierenden Polymergebilde mit einer mindestens ein Anionen und ein Polykation umfassenden Verbindung,
iii) gegebenenfalls Nachvernetzung des wasserabsorbierenden Polymergebildes, wobei der Verfahrensschritt iii) vor, während oder nach dem Verfahrensschritt ii) durchgeführt werden kann.

"Unbehandelt" im Sinne der vorliegenden Erfindung bedeutet, dass die wasserabsorbierenden Polymergebilde noch nicht mit der Verbindung umfassend mindestens ein Anionen und ein Polykation der Struktur I in Kontakt gebracht und auch noch nicht oberflächennachvernetzt worden sind. Die Bezeichnung "unbehandelt" schließt hingegen nicht aus, dass die wasserabsorbierenden Polymergebilde mittels anderer Oberflächenmodifizierungsmaßnahmen, wie etwa der Beschichtung mit Siliziumdioxid oder Kieselsäure zum Zwecke der Erhöhung der Permeabilität der Polymergebilde im gequollenen Zustand oder der Oberflächenbehandlung mit Alumiriiumsalzen modifiziert sein können.

Als unbehandelte, wasserabsorbierende Polymergebilde werden dabei im Verfahrensschritt i) vorzugsweise Polymere bereitgestellt, welche erhalten wurden durch ein Verfahren umfassend die Verfahrensschritte:

a) radikalische Polymerisation säuregruppen-tragender, ethylenisch ungesättigter, gegebenenfalls teilneutralisierter Monomere in Gegenwart eines Vernetzers unter Bildung eines Hydrogels;

b) gegebenenfalls Zerkleinern des Hydrogels;

c) zumindest teilweises Trocknen des gegebenenfalls zerkleinerten Hydrogels unter Erhalt wasserabsorbierender Polymergebilde;

d) gegebenenfalls Zermahlen des so erhaltenen absorbierenden Polymergebildes und absieben auf eine gewünschte Partikelgrößenfraktion;

e) gegebenenfalls weitere Oberflächenmodifizierungen der so erhalten wasserabsorbierenden Polymergebilde.

Die im Verfahrensschritt a) erfolgende radikalische Polymerisation erfolgt vorzugsweise in wässriger Lösung, wobei diese wässrige Lösung neben Wasser als Lösungsmittel vorzugsweise

(α1) die ethylenisch ungesättigten, säuregruppen-tragenden Monomeren oder deren Salze, wobei Acrylsäure als säuregruppentragendes Monomer besonders bevorzugt ist,
(α2) gegebenenfalls monoethylenisch ungesättigte, mit (α1) copolymerisierbare Monomere,
(α3) den Vernetzer,
(α5) gegebenenfalls ein wasserlösliches Polymer, sowie
(α7) gegebenenfalls einen oder mehrere Hilfsmittel.

Als monoethylenisch ungesättigte, mit (α1) copolymerisierbare Monomere, wasserlösliche Polymere und Hilfsmittel sind wiederum diejenigen Verbindungen bevorzugt, die bereits eingangs im Zusammenhang mit den erfindungsgemäßen Polymergebilden als mit ($\alpha$1) copolymerisierbare Monomere, als wasserlösliche Polymere bzw., als Hilfsmittel genannt wurden.

Aus den vorgenannten Monomeren, Comonomeren, Vernetzer, wasserlöslichen Polymeren und Hilfsstoffen lassen sich die wasserabsorbierende Polymergebilde durch verschiedene Polymerisationsweisen herstellen. Beispielsweise sind in diesem Zusammenhang Massepolymerisation, die vorzugsweise in Knetreaktoren wie Extrudern erfolgt, Lösungspolymerisation, Spraypolymerisation, inverse Emulsionspolymerisation und inverse Suspensionspolymerisation zu nennen.

Bevorzugt wird die Lösungspolymerisation in Wasser als Lösungsmittel durchgeführt. Die Lösungspolymerisation kann kontinuierlich oder diskontinuierlich erfolgen. Aus dem Stand der Technik ist ein breites Spektrum von Variationsmöglichkeiten hinsichtlich Reaktionsverhältnisse wie Temperaturen, Art und Menge der Initiatoren als auch der Reaktionslösung zu entnehmen. Typische Verfahren sind in den folgenden Patentschriften beschrieben: US 4,286,082, DE 27 06 135, US 4,076,663, DE 35 03 458, DE 40 20 780; DE 42 44 548, DE 43 23 001, DE 43 33 056, DE 44 18 818. Die Offenbarungen werden hiermit als Referenz eingeführt und gelten somit als Teil der Offenbarung.

Die Polymerisation wird wie allgemein üblich durch einen Initiator ausgelöst. Als Initiatoren zur Initiierung der Polymerisation können alle unter den Polymerisationsbedingungen Radikale bildende Initiatoren verwendet werden, die üblicherweise bei der Herstellung von Superabsorbern eingesetzt werden. Auch eine Initiierung der Polymerisation durch Einwirkung von Elektronenstrahlen auf die polymerisierbare, wässrige Mischung ist möglich. Die Polymerisation kann allerdings auch in Abwesenheit von Initiatoren der oben genannten Art durch Einwirkung energiereicher Strahlung in Gegenwart von Photoinitiatoren ausgelöst werden. Polymerisationsinitiatoren können in einer Lösung erfindungsgemäßer Monomere gelöst oder dispergiert enthalten sein. Als Initiatoren kommen sämtliche dem Fachmann bekannte in Radikale zerfallende Verbindungen in Betracht. Hierunter fallen insbesondere diejenigen Initiatoren, die bereits in der WO 2004/037903 A2 als mögliche Initiatoren genannt werden.

Besonders bevorzugt wird zur Herstellung der wasserabsorbierenden Polymergebilde ein Redoxsystem bestehend aus Wasserstoffperoxid, Natriumperoxodisulfat und Ascorbinsäure eingesetzt.

Auch die inverse Suspensions- und Emulsionspolymerisation kann zur Herstellung der Polymergebilde angewendet werden. Gemäß diesen Prozessen wird eine wässrige, teilneutralisierte Lösung der Monomeren ($\alpha$1), und ($\alpha$2), gegebenenfalls beinhaltend wasserlösliche Polymere und Hilfsstoffe, mit Hilfe von Schutzkolloiden und/oder Emulgatoren in einem hydrophoben, organischen Lösungsmittel dispergiert und durch Radikalinitiatoren die Polymerisation gestartet. Die Vernetzer sind entweder in der Monomerlösung gelöst und werden mit dieser zusammen dosiert oder aber separat und gegebenenfalls während der Polymerisation zugefügt. Gegebenenfalls erfolgt die Zugabe eines wasserlöslichen Polymeren ($\alpha$4) als Pfropfgrundlage über die Monomerlösung oder durch direkte Vorlage in die Ölphase. Anschließend wird das Wasser azeotrop aus dem Gemisch entfernt und das Polymerisat abfiltriert.

Weiterhin kann sowohl bei der Lösungspolymerisation als auch bei der inversen Suspensions- und Emulsionspolymerisation die Vernetzung durch Einpolymerisation des in der Monomerlösung gelösten polyfunktionellen Vernetzers und/oder durch Reaktion geeigneter Vernetzer mit funktionellen Gruppen des Polymeren während der Polymerisationsschritte erfolgen. Die Verfahren sind beispielsweise in den Veröffentlichungen US 43 40 706, DE 37 13 601, DE 28 40 010 und WO 96/05234 A1 beschrieben, deren entsprechende Offenbarung hiermit als Referenz eingeführt wird.

Die bei der Lösungspolymerisation oder der inversen Suspensions- und Emulsionspolymerisation im Verfahrensschritt a) erhaltenen Hydrogele werden im Verfahrensschritt c) zumindest teilweise getrocknet.

Insbesondere im Falle der Lösungspolymerisation ist es jedoch bevorzugt, dass die Hydrogele vor der Trocknung zunächst in einem zusätzlichen Verfahrensschritt b) zerkleinert werden. Dieses Zerkleinern erfolgt durch dem Fachmann bekannte Zerkleinerungsvorrichtungen, wie etwa einem Fleischwolf.

Die Trocknung des Hydrogels erfolgt vorzugsweise in geeigneten Trocknern oder Öfen. Beispielhaft seien Drehrohröfen, Wirbelbetttrockner, Tellertrockner, Paddeltrockner oder Infrarottrockner genannt. Weiterhin ist es erfindungsgemäß bevorzugt, dass die Trocknung des Hydrogels im Verfahrensschritt c) bis zu einem Wassergehalt von 0,5 bis 50 Gew.-%, vorzugsweise von 1 bis 25 Gew.-%, besonders bevorzugt 2 bis 10 Gew.-% erfolgt, wobei die Trocknungstemperaturen üblicherweise in einem Bereich von 100 bis 200°C liegen.

Die im Verfahrensschritt c) erhaltenen, zumindest teilweise getrockneten wasserabsorbierenden Polymergebilde können, insbesondere dann, wenn sie durch Lösungspolymerisation erhalten wurden, in einem weiteren Verfahrensschritt d) noch zermahlen und auf die eingangs genannte Wunschkomgröße abgesiebt werden. Das Zermahlen der getrockneten, wasserabsorbierenden Polymergebilde erfolgt vorzugsweise in geeigneten, mechanischen Zerkleinerungsvorrichtungen, wie etwa einer Kugelmühle.

Im Anschluss an die Trocknung der Hydrogele und der gegebenenfalls erfolgten weiteren Konfektionierung der getrockneten wasserabsorbierenden Polymergebilde können diese in einem weiteren Verfahrenschritt e) im Oberflachenbereich modifiziert werden (ausgenommen von der im Verfahrensschritt e) durchgefiihrten Oberflächenmodifizierung ist die im nachfolgenden noch eingehender beschriebene Modifizierung mit der Verbindung umfassend mindesten ein Anion und ein Polykation und sowie die Oberflächennachvernetzung).

Als bevorzugte Modifizierungsmaßnahme sei hier das in Kontakt bringen des Aussenbereiches der Polymergebilde mit einer Verbindung enthaltend $A1^{3+}$-Ionen vor, während oder nach, vorzugsweise nach, dem in Kontakt bringen mit dem Nachvernetzer bzw. dem Fluid beinhaltend den Nachvernetzer zu nennen. Dabei ist es bevorzugt, dass die Verbindung enthaltend $A1^{3+}$-Ionen in einer Menge in einem Bereich von 0,01 bis 30 Gew. -%, besonders bevorzugt in einer Menge in einem Bereich von 0,1 bis 20 Gew. -% und darüber hinaus bevorzugt in einer Menge in einem Bereich von 0,3 bis 5 Gew. -%, jeweils bezogen auf das Gewicht der Polymergebilde, mit den Polymergebilden in Kontakt gebracht wird.

Das in Kontakt bringen des Aussenbereiches der Polymergebilde mit der $A1^{3+}$-Ionen enthaltenden Verbindung erfolgt vorzugsweise dadurch, dass das Polymergebilde mit der Verbindung unter trockenen Bedingungen vermischt wird oder aber dadurch, dass die Polymergebilde mit einem Fluid $F_1$ umfassend ein Lösemittel, vorzugsweise Wasser, mit Wasser mischbare organische Lösemittel wie etwa Methanol oder Ethanol oder Mischungen aus mindestens zwei davon, sowie die $A1^{3+}$-Ionen enthaltende Verbindung in Kontakt gebracht werden, wobei das in Kontakt bringen vorzugsweise durch Besprühen der Polymerteilchen mit dem Fluid $F_1$ und Vermischen erfolgt. In diesem Zusammenhang ist es weiterhin bevorzugt, dass das in Kontakt bringen der Polymergebilde mit dem Fluid $F_1$ enthaltend die $Al^{3+}$-Ionen enthaltende Verbindung in einem zweistufigen Verfahren erfolgt. Dabei umfasst das zweistufige Verfahren einen ersten Mischvorgang, bei dem eine Vielzahl von Polymergebilden mit dem Fluid $F_1$ vermischt wird, und einem zweiten Mischvorgang, bei dem das Fluid $F_1$ im Inneren der Polymergebilde homogenisiert wird, wobei die Polymergebilde in dem ersten Mischvorgang mit einer Geschwindigkeit gemischt werden, dass die Bewegungsenergie der einzelnen Polymergebilde im Mittel größer ist als die Haftungsenergie zwischen den einzelnen Polymergebilden, und die Polymergebilde in dem zweiten Mischvorgang mit einer geringeren Geschwindigkeit als im ersten Mischvorgang durchmischt werden.

Durch die Behandlung der Polymergebilde mit dem Fluid $F_1$ beinhaltend die $Al^{3+}$-Ionen enthaltende Verbindung durch das vorstehend beschriebene, zweistufige Verfahren können Polymergebilde mit verbesserten Absorptionseigenschaften erhalten werden.

Vorzugsweise ist dabei die $Al^{3+}$-Ionen enthaltende Verbindung ohne Berücksichtigung von Kristallwasser in einer Menge in einem Bereich von 0,1 bis 50 Gew. - %, besonders bevorzugt in einer Menge in einem Bereich von 1 bis 30 Gew.-%, jeweils bezogen auf das Gesamtgewicht des Fluids $F_1$, in dem Fluid $F_1$ enthalten.

Es ist weiterhin bevorzugt, dass das Fluid $F_1$ in einer Menge in einem Bereich von 0,01 bis 15 Gew.-%, besonders bevorzugt in einer Menge in einem Bereich von 0,05 bis 6 Gew.-%, jeweils bezogen auf das Gewicht der Polymergebilde, mit den Polymergebilden in Kontakt gebracht wird.

Bevorzugte $Al^{3+}$-Ionen enthaltenden Verbindungen sind $AlCl_3 \times 6H_2O$, $NaAl(SO_4)_2$ x 12 $H_2O$, $KAl(SO_4)_4$ x 12 $H_2O$ oder$Al_2(SO_4)_3$ x 14-18 $H_2O$.

Als weitere Oberflächenmodifizierungsmaßnahme sei an dieser Stelle das in Kontakt bringen der wasserabsorbierenden Polymergebilde mit anorganischen Partikeln, beispielsweise mit feinteiligem Siliziumdioxid, welches vorzugsweise in wässriger Suspension appliziert wird, oder mit Kieselsäuresol erwähnt.

Die vorstehend im Verfahrensschritt e) erfolgenden Modifizierungsmaßnahmen, insbesondere die Behandlung mit einer $A1^{3+}$-Ionen enthaltenden Verbindung, können grundsätzlich auch während der Verfahrensschritte ii) und iii) oder auch nach Durchführung der Verfahrensschritte ii) oder iii) erfolgen, wobei die Anwendung dieser Modifizierungsmaßnahmen nach Durchführung der Verfahrensschritte ii) und iii) besonders bevorzugt ist.

Im Verfahrensschritt ii) des erfindungsgemäßen Verfahrens werden die unbehandelten, wasserabsorbierenden Polymergebilde mit einer Verbindung beinhaltend mindestens ein Anion und ein Polykation in Kontakt gebracht. Dabei ist es erfindungsgemäß bevorzugt, dass es sich bei dem Polykation um ein Polykation der Struktur I

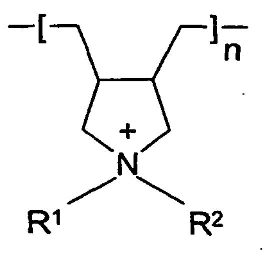

Struktur I

handelt, in der

$R^1$ und $R^2$ gleich oder verschieden sein können und ein Kohlenwasserstoffrest mit 1 bis 10 Kohlenstoffatomen, vorzugsweise 1 bis 7 Kohlenstoffatomen, besonders bevorzugt 1 bis 4 Kohlenstoffatomen, darüber hinaus bevorzugt 1 bis 2 Kohlenstoffatomen und am meisten bevorzugt eine Methylgruppe darstellen und

n einen Wert von mindestens 25, vorzugsweise mindestens 31, noch mehr bevorzugt mindestens 100, darüber hinaus bevorzugt mindestens 1.000, noch mehr bevorzugt mindestens 2.000, darüber hinaus noch mehr bevorzugt mindestens 2.500, noch mehr bevorzugt mindestens 3.000 und am meisten bevorzugt mindestens 5.000 hat.

Vorzugsweise weist die Verbindung beinhaltend mindestens ein Anion und ein Polykation ein Molekulargewicht von mehr als 3.000 g/mol, vorzugsweise mehr als 4.000 g/mol, noch mehr bevorzugt mehr als 5.000 g/mol, darüber hinaus noch mehr bevorzugt mehr als 7.000 g/mol und am meisten bevorzugt mehr als 10.000 g/mol auf.

Besonders bevorzugt handelt es sich bei dem Polykation um ein Polydiallyldimethylamoniumchlorid mit einem Wert für n von mindestens 25, vorzugsweise mindestens 31, darüber hinaus bevorzugt mindestens 100, noch mehr bevorzugt mindestens 1.000, weiter bevorzugt mindestens 2.500 und am meisten bevorzugt mindestens 5.000.

Weiterhin kann gemäß dem erfindungsgemäßen Verfahren im Verfahrensschritt ii) die Verbindung beinhaltend das mindestens eine Anion und das Polykation direkt oder aber mittels eines weiteren Fluids $F_2$ umfassend ein Lösungsmittel, vorzugsweise Wasser, mit Wasser mischbare organische Lösemittel wie etwa Methanol oder Ethanol oder Mischungen aus mindestens zwei davon sowie diese Verbindung mit dem unbehandelten wasserabsorbierenden Polymergebilde in Kontakt gebracht werden.

Weiterhin ist es erfindungsgemäß bevorzugt, dass das unbehandelte wasserabsorbierende Polymergebilde mit dieser Verbindung in einer Menge in einem Bereich von 0,001 bis 10 Gew.-%, vorzugsweise 0,01 bis 7,5 Gew.-% und noch mehr bevorzugt mindestens 0,5 bis 5 Gew.-%, bezogen auf das Gewicht des wasserabsorbierenden Polymergebildes, in Kontakt gebracht wird. Sofern diese Verbindung in Form eines Fluids $F_2$ eingesetzt wird, ist es weiterhin bevorzugt, dass Verbindung in dem Fluid $F_2$ in Form einer 10 bis 80 gew.-%igen, vorzugsweise einer 20 bis 70 gew.-%igen, ganz besonders bevorzugt einer 30 bis 60 gew.-%igen Lösung, vorzugsweise wässrigen Lösung, mit dem unbehandelten wasserabsorbierenden Polymergebilde in Kontakt gebracht wird.

Geeignete Mischaggregate zum Aufbringen des Fluids $F_2$ sind wiederum der Patterson-Kelley-Mischer, DRAIS-Turbulenzmischer, Lödigemischer, Ruberg-Mischer, Schneckenmischer, Tellermischer und Wirbelschichtmischer sowie kontinuierlich arbeitende senkrechte Mischer, in denen das Polymergebilde mittels rotierender Messer in schneller Frequenz gemischt wird (Schugi-Mischer).

Im Verfahrensschritt iii) erfolgt gegebenenfalls die Oberflächennachvernetzung des wasserabsorbierenden Polymergebildes, bei der die Polymergebilde zunächst mit einem Oberflächennachvernetzer in Kontakt gebracht und erhitzt werden. Dabei wird der Nachvernetzer insbesondere dann, wenn er unter den Nachvemetzungsbedingungen nicht flüssig ist, vorzugsweise in Form eines Fluids $F_3$ umfassend den Nachvernetzer sowie ein Lösungsmittel mit den Polymerteilchen in Kontakt gebracht. Als Lösungsmittel werden dabei vorzugsweise Wasser, mit Wasser mischbare organische Lösungsmittel wie etwa Methanol, Ethanol, 1-Propanol, 2-Propanol oder 1-Butanol oder Mischungen aus mindestens zwei dieser Lösungsmittel eingesetzt, wobei Wasser als Lösungsmittel am meisten bevorzugt ist. Weiterhin ist es bevorzugt, dass der Nachvernetzer in dem Fluid $F_3$ in einer Menge in einem Bereich von 5 bis 75 Gew.-%, besonders bevorzugt 10 bis 50 Gew.-% und am meisten bevorzugt 15 bis 40 Gew.-%, bezogen auf das Gesamtgewicht des Fluids $F_3$, enthalten ist.

Das in Kontakt bringen des Polymergebildes mit dem Fluid $F_3$ beinhaltend den Nachvernetzer erfolgt im erfindungsgemäßen Verfahren vorzugsweise durch gutes Vermischen des Fluids $F_3$ mit dem Polymergebilde.

Geeignete Mischaggregate zum Aufbringen des Fluids $F_3$ sind wiederum z. B. der Patterson-Kelley-Mischer, DRAIS-Turbulenzmischer, Lödigemischer, Ruberg-Mischer, Schneckenmischer, Tellermischer und Wirbelschichtmischer sowie kontinuierlich arbeitende senkrechte Mischer, in denen das Polymergebilde mittels rotierender Messer in schneller Frequenz gemischt wird (Schugi-Mischer).

Das Polymergebilde wird in dem erfindungsgemäßen Verfahren bei der Nachvernetzung vorzugsweise mit höchstens 20 Gew. -%, besonders bevorzugt mit höchstens 15 Gew.-%, darüber hinaus bevorzugt mit höchstens 10 Gew. -%, darüber hinaus noch mehr bevorzugt mit höchstens 5 Gew. -% an Lösungsmittel, vorzugsweise Wasser, in Kontakt gebracht und am allermeisten bevorzugt mit weniger als 3 Gew.-%, jeweils bezogen auf das Gewicht des Polymergebildes.

Bei Polymergebilden in der Form von vorzugsweise kugelförmigen Teilchen ist es erfindungsgemäß weiterhin bevorzugt, dass das in Kontakt bringen derart erfolgt, dass lediglich der Aussenbereich, nicht jedoch der innere Bereich der teilchenförmigen Polymergebilde mit dem Fluid $F_3$ und somit dem Nachvernetzer in Kontakt gebracht werden.

Als Nachvernetzer, die im erfindungsgemäßen Verfahren eingesetzt werden, werden vorzugsweise Verbindungen verstanden, die mindestens zwei funktionelle Gruppen aufweisen, die mit funktionellen Gruppen eines Polymergebildes in einer Kondensationsreaktion (=Kondensationsvemetzer), in einer Additionsreaktion oder in einer Ringöffnungsreaktion reagieren können oder aber polyvalente Metallkationen, die mittels elektrostatischer Wechselwirkung

zwischen dem polyvalenten Metallkation und den funktionellen Gruppen eines Polymergebildes eine Vernetzung des Polymergebildes ermöglichen. Als Nachvernetzer sind im erfindungsgemäßen Verfahren diejenigen bevorzugt, die in WO 2004/037903 A2 als Vernetzer der Vernetzerklassen II und IV genannt wurden.

Unter diesen Verbindungen sind als Nachvernetzer besonders bevorzugt Kondensationsvemetzer wie beispielsweise Diethylenglykol, Triethylenglykol, Polyethylenglykol, Glyzerin, Polyglyzerin, Propylenglykol, Diethanolamin, Triethanolamin, Polyoxypropylen, Oxyethylen-Oxypropylen-Blockcopolymere, Sorbitanfettsäureester, Polyoxyethylensorbitanfettsäureester, Trimethylolpropan, Pentaerytrit, Polyvinylalkohol, Sorbitol, 1, 3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-l,3-dioxan-2-on sowie 1,3-Dioxolan-2-on.

Nachdem die Polymergebilde mit dem Nachvernetzer bzw. mit dem Fluid beinhaltend den Nachvernetzer in Kontakt gebracht wurden, werden sie auf eine Temperatur im Bereich von 50 bis 300°C, vorzugsweise 75 bis 275°C und besonders bevorzugt 150 bis 250°C erhitzt, so dass, vorzugsweise wodurch, der Aussenbereich der Polymergebilde im Vergleich zum Innenbereich stärker vernetzt wird (=Nachvernetzung). Die Zeitdauer der Wärmebehandlung wird durch die Gefahr, dass das gewünschte Eigenschaftsprofil der Polymergebilde infolge von Hitzeeinwirkung zerstört wird, begrenzt.

Das in Kontakt bringen der unbehandelten wasserabsorbierenden Polymergebilde mit der Verbindung umfassend mindestens ein Anion und ein Polykation bzw. mit dem Fluid $F_2$ beinhaltend diese Verbindung im Verfahrensschritt ii) kann zu verschiedenen Zeitpunkten des erfindungsgemäßen Verfahrens erfolgen:

1. Die Verbindung umfassend mindestens ein Anion und ein Polykation bzw. das Fluid $F_2$ können mit dem im Verfahrensschritt b) erhaltenen Polymergel in Kontakt gebracht werden, welches vorzugsweise noch einen Wassergehalt von mindestens 10 Gew.-%, vorzugsweise mindestens 20 Gew.% und am meisten bevorzugt mindestens 30 Gew.-% aufweist und erst anschließend erfolgt eines Oberflächennachvemetzung gemäß dem Verfahrensschritt iii).

2. Die Verbindung umfassend mindestens ein Anion und ein Polykation bzw. das Fluid $F_2$ können mit dem im Verfahrensschritt b) gegebenenfalls zerkleinerten Polymergel in Kontakt gebracht werden, wobei auch dieses vorzugsweise noch einen Wassergehalt von mindestens 10 Gew.-%, vorzugsweise mindestens 20 Gew.-% und am meisten bevorzugt mindestens 30 Gew.-% aufweist. Vor, während oder nach dem in Kontakt bringen mit der Verbindung erfolgt die Oberflächennachvemetzung gemäß dem Verfahrensschritt iii).

3. Die Verbindung umfassend mindestens ein Anion und ein Polykation bzw. das Fluid $F_2$ können mit dem im Verfahrensschritt c) erhaltenen getrockneten wasserabsorbierenden Polymergebilden in Kontakt gebracht werden. Vor, während oder nach dem in Kontakt bringen mit der Verbindung erfolgt die Oberflächennachvemetzung gemäß dem Verfahrensschritt iii).

4. Die Verbindung umfassend mindestens ein Anion und ein Polykation bzw. das Fluid $F_2$ können mit dem im Verfahrensschritt d) erhaltenen, zermahlenen wasserabsorbierenden Polymergebilden in Kontakt gebracht werden. Vor, während oder nach dem in Kontakt bringen mit der Verbindung erfolgt die Oberflächennachvemetzung gemäß dem Verfahrensschritt iii).

5. Die Verbindung umfassend mindestens ein Anion und ein Polykation bzw. das Fluid $F_2$ können mit dem im Verfahrensschritt e) erhaltenen, oberflächenmodifizierten wasserabsorbierenden Polymergebilden in Kontakt gebracht werden. Vor, während oder nach dem in Kontakt bringen mit der Verbindung erfolgt die Oberflächennachvernetzung gemäß dem Verfahrensschritt iii).

In einer besonders bevorzugten Ausführungsform des erfindungsgemäßen Verfahrens werden die Verfahrensschritte ii) und iii) zeitgleich durchgeführt. In diesem Fall wird die Verbindung umfassend mindestens ein Anion und ein Polykation bzw. das Fluid $F_2$ in Kombination mit dem $F_3$ eingesetzt, wobei auch ein gemeinsames Fluid beinhaltend die Verbindung umfassend mindestens ein Anion und ein Polykation sowie einen Nachvernetzer eingesetzt werden kann. Nachdem die Polymergebilde mit dem Nachvernetzer und mit der Verbindung umfassend mindestens ein Anion und ein Polykation zeitgleich in den Verfahrensschritten ii) und iii) in Kontakt gebracht worden ist, erfolgt die Nachvernetzung durch Erhitzen der Polymergebilde auf die vorstehend genannten Nachvemetzungstemperaturen.

Jede dieser Varianten 1. bis 5. stellt eine besondere Ausführungsform des erfindungsgemäßen Verfahrens dar, wobei die Variante 4. am meisten bevorzugt ist. Hierbei ist es weiterhin bevorzugt, dass bei der Variante 4. im Anschluss an die Oberflächennachvernetzung noch eine Modifizierung mittels einer $A1^{3+}$-Ionen enthaltenden Verbindung gemäß dem vorstehend beschriebenen Verfahrensschritt e) erfolgt.

Weiterhin kann es gemäß einer besonderen Ausgestaltung des erfindungsgemäßen Verfahrens bevorzugt sein, die durch die vorstehend beschriebenen Verfahrensvarianten erhaltenen, oberflächennachvernetzten und mit dem mindestens einen Anion und dem Polykation in Kontakt gebrachten wasserabsorbierenden Polymergebilde in einem weiteren Verfahrensschritt iv) mit einer Feinstteilchenkomponente in Kontakt zu bringen und anschließend die Feinstteilchen auf der Oberfläche der wasserabsorbierenden Polymergebilde zu immobilisieren. Diese besondere Ausgestaltung ist insbesondere dann bevorzugt, wenn das wasserabsorbierende Polymergebilde noch nicht vor oder während des Verfahrensschrittes ii) und/oder iii) mit $A1^{3+}$-Ionen oder anorganischen Partikeln in Kontakt gebracht worden ist.

Bei den Feinstteilchen kann es sich um ein organisches oder um ein anorganisches Feinstteilchen handeln, wobei ein anorganisches Feinstteilchen besonders bevorzugt ist.

Weiterhin kann es sich gemäß einer besonderen Ausführungsform des erfindungsgemäßen Verfahrens bei den Feinstteilchen um wasserlösliche Feinstteilchen handeln, wobei unter wasserlöslichen Feinstteilchen vorzugsweise Feinstteilchen verstanden werden, von welchen bei 25°C mindestens 1 g, vorzugsweise mindestens 5 g und am meisten bevorzugt mindestens 10 g in 100 ml Wasser gelöst werden können.

Gemäß einer anderen besonderen Ausführungsform des erfindungsgemäßen Verfahrens handelt es sich bei den Feinstteilchen um wasserunlösliche Feinstteilchen, wobei unter wasserunlöslichen Feinstteilchen vorzugsweise Feinstteilchen verstanden werden, von welchen bei 25°C weniger als 1 g, vorzugsweise weniger als 0,1 g und am meisten bevorzugt weniger als 0,01 g in 100 ml Wasser gelöst werden können.

Weiterhin ist es erfindungsgemäß bevorzugt, dass das vorzugsweise anorganische Feinstteilchen ein mindestens zweiwertiges, vorzugsweise mindestens dreiwertiges Metall aufweist. Bevorzugte, mindestens zweiwertige Metalle sind ausgewählt aus der Gruppe umfassend Beryllium, Magnesium, Kalzium, Barium, Strontium, Aluminium, Bor, Zirkonium, Silizium, Scandium, Vanadium, Cer, Yttrium, Lanthan, Niob, Chrom, Molybdän, Mangan, Palladium, Platin, Cadmium, Quecksilber, Eisen, Kupfer, Zink, Titan, Kobalt oder Nickel, wobei Aluminium am meisten bevorzugt ist.

Weiterhin ist es erfindungsgemäß bevorzugt, dass das vorzugsweise anorganische Feinstteilchen als Salz umfassend das mindestens zweiwertige Metall in Form eines mindestens zweiwertigen Kations $K^{n+}$ (mit $n \geq 2$) und mindestens ein Anion $A^{m-}$ (mit $m \geq 1$) vorliegt.

Erfindungsgemäß besonders bevorzugte Feinstteilchen sind ausgewählt aus der Gruppe umfassend Aluminiumsalze, wie etwa Aluminiumchlorid, Polyaluminiumchlorid, Aluminiumsulfat, Aluminiumnitrat, bis-Aluminium-Kalium-Sulfat, bis-Aluminium-Natrium-Sulfat, Aluminiumlactat, Aluminiumoxalat, Aluminiumcitrat, Aluminiumglyoxylat, Aluminiumsuccinat, Aluminiumitaconat, Aluminiumcrotonat, Aluminiumbutyrat, Aluminiumsorbat, Aluminiummalonat, Aluminiumbenzoat, Aluminiumtartrat, Aluminiumpyruvat, Aluiniumvalerat, Aluminiumformat, Aluminiumglutarat, Aluminiumpropanat oder Aluminiumacetat, Phosphate der Formel $M_4P_2O_7$, $M_2HPO_4$ oder $M_3PO_4$, worin M für ein Äquivalent eines unter Kalzium, Magnesium, Strontium, Barium, Zink, Eisen, Aluminium, Titan, Zirkonium, Hafnium, Zinn, Cer, Scandium, Yttrium oder Lanthan oder Gemischen davon ausgewählten Metalls steht, wie etwa Calciumhydrogenphosphat, tertiäres Calciumphosphat, Apatit, Thomasmehl der Formel $Ca_5(PO_4)[SiO_4]$, Berlinit der Formel $AlPO_4$ oder Rhenaniaphosphat der Formel 3 $CaNaPO_4Ca_2SiO_4$, Kalziumchlorid, Kalziumnitrat, Magnesiumchlorid, Magnesiumsulfat, Magnesiumnitrat, Zinkchlorid, Zinksulfat, Zinknitrat, Kupfersulfat, Kobaltchlorid, Zirkoniumchlorid, Zirkoniumsulfat, Zirkoniumnitrat; Siliziumdioxide wie etwa Aerosil® oder Titandioxide. Eine weitere bevorzugte Verbindung, die als Salz aufgefasst wird, ist Al(O)OH..

Am meisten bevorzugte Feinstteilchen sind Aluminiumsalze ausgewählt aus der Gruppe umfassend $AlCl_3 \times 6H_2O$, $NaAl(SO_4)_2 \times 12\ H_2O$ $KAl(SO_4)_2 \times 12\ H_2O$, $Al_2(SO_4)_3 \times$ 14-18 $H_2O$, Aluminiumlactat oder Aluminiumcitrat, wobei $Al_2(SO_4)_3 \times$ 14-18 $H_2O$ darüber hinaus besonders bevorzugt ist.

Gemäß einer besonderen Ausführungsform des erfindungsgemäßen Verfahrens beinhaltet die Feinstteilchenkomponente mindestens zwei verschiedene Feinstteilchensorten, beispielsweise ein Aluminiumsalz und ein von einem Aluminiumsalz verschiedenes Salz oder aber zwei verschiedene Aluminiumsalze.

Weiterhin ist es erfindungsgemäß bevorzugt, dass mindestens 50 Gew.-%, besonders bevorzugt mindestens 75 Gew.-%, darüber hinaus noch mehr bevorzugt mindestens 95 Gew.-% und am meisten bevorzugt mindestens 99 Gew.-% der Feinstteilchen einen mittleren Teilchendurchmesser (Gewichtsmittel) in einem Bereich von 10 bis 1.000 $\mu$m, vorzugsweise von 50 $\mu$m bis 800 $\mu$m, besonders bevorzugt von 100 bis 600 $\mu$m und am meisten bevorzugt von 200 bis 400 $\mu$m aufweisen, jeweils bestimmt durch dem Fachmann bekannte Verfahren zur Partikelgrößenbestimmung, beispielsweise durch Siebanalyse oder mittels eines Coulter-Counters.

Gemäß einer besonderen Ausführungsform des erfindungsgemäßen Verfahrens beträgt der Anteil an Feinstteilchen mit einer mittleren Partikelgröße > 150 $\mu$m mehr als 20 Gew.%, besonders bevorzugt mehr als 30 Gew.-% und darüber hinaus bevorzugt mehr als 40 Gew.-%, jeweils bezogen auf das Gesamtgewicht der Feinstteilchen.

Gemäß einer besonders bevorzugten Ausführungsform des erfindungsgemäßen Verfahrens beinhaltet die Feinstteilchenkomponente zusätzlich einen Binder, wobei als Binder vorzugsweise organische Verbindungen eingesetzt werden. Besonders bevorzugt ist es, dass die organische Verbindung ein vorzugsweise lineares Polymer ist, vorzugsweise ein lineares Polymer ausgewählt aus der Gruppe umfassend Polyurethane, Polyester, Polyamide, Polyesteramide, Polyolefine, Polyvinylester, Polyether, Polystyrole, Polyimide, insbesondere Polyetherimide, Polyimine, Schwefelpolymere, insbesondere Polysulfon, Polyacetale, insbesondere Polyoxymethylene, Fluorkunststoffe, insbesondere Polyvinylidenfluorid, Styrol-Olefin-Copolymere, Polyacrylate, Ethylen-Vinylacetat-Copolymere oder Gemische aus zwei oder mehr der genannten Polymere, wobei unter diesen Polymeren Polykondensate und unter diesen Polyether besonders bevorzugt und lineare Polyether am meisten bevorzugt sind.

Besonders geeignete lineare Polyether umfassen Polyalkylenglykole, insbesondere Polyethylenglykole, Polypropylenglykole, Poly(ethylen/propylen)glykole mit statistischer oder blockartiger Anordnung der Ethylen- oder Propylen-Monomere oder Mischungen aus mindesten zwei dieser Polyalkylenglykole.

Weitere geeignete, vorzugsweise lineare Polymere sind diejenigen Polymere, die in der DE-A-103 34 286 als "thermoplastische Klebstoffe" genannt werden. Der Offenbarungsgehalt der DE-A-103 34 286 hinsichtlich thermoplastischer Klebstoffe wird hiermit als Referenz eingeführt und bildet einen Teil der Offenbarung der vorliegenden Erfindung.

Weiterhin ist es erfindungsgemäß bevorzugt, dass die organischen Verbindung als Binderhauptkomponente ein Gewichtsmittel des Molekulargewichts $M_w$ in einem Bereich von 100 bis 1.000.000 g/mol, besonders bevorzugt in einem Bereich von 1.000 bis 100.000 g/mol und am meisten bevorzugt in einem Bereich von 5.000 bis 20.000 g/mol aufweist.

Weiterhin ist es bevorzugt, dass der Binder in partikulärer Form vorliegt, wobei in diesem Zusammenhang als partikulärer Binder insbesondere partikuläre Polyalkylenglykole, wie etwa partikuläre Polyethylenglykole oder Polypropylenglykole, vorteilhaft sind, wobei es besonders bevorzugt ist, dass der partikuläre Binder zu mindestens 50 Gew.-%, besonders bevorzugt zu mindestens 75 Gew.-%, darüber hinaus noch mehr bevorzugt zu mindestens 95 Gew.-% und am meisten bevorzugt zu mindestens 99 Gew.-% auf Partikel mit einem mittleren Teilchendurchmesser (Gewichtsmittel) von weniger als 500 $\mu$m, vorzugsweise weniger als 400 $\mu$m, besonders bevorzugt weniger als 300 $\mu$m und am meisten bevorzugt weniger als 150 $\mu$m basiert, jeweils bestimmt durch dem Fachmann bekannte Verfahren zur Partikelgrößenbestimmung, beispielsweise durch Siebanalyse oder mittels eines Coulter-Counters.

Die Menge an Feinstteilchen, die im Verfahrensschritt iv) mit dem wasserabsorbierenden Polymergebilde in Kontakt gebracht werden, liegt vorzugsweise in einem Bereich von 0,001 bis 10 Gew.-%, besonders bevorzugt in einem Bereich von 0.01 bis 5 Gew.-% und am meisten bevorzugt in einem Bereich von 0,1 bis 2 Gew.-%, jeweils bezogen auf das Gewicht der wasserabsorbierenden Polymergebilde, während das Gewicht des Bindemittels, sofern eingesetzt, vorzugsweise in einem Bereich von 0,0001 bis 5 Gew.-% und besonders bevorzugt in einem Bereich von 0,001 bis 2 Gew.-%, jeweils bezogen auf das Gewicht der wasserabsorbierenden Polymergebilde, liegt. Das Gewichtsverhältnis zwischen Feinstteilchen und Bindemittel liegt vorzugsweise in einem Bereich von Feinstteilchen : Bindemittel von 20 : 1 bis 1 : 20, besonders bevorzugt von 10 : 1 bis 1 : 10 und am meisten bevorzugt von 10 : 1 bis 2 : 1.

Das in Kontakt bringen der Feinstteilchenkomponente mit dem wasserabsorbierenden Polymergebilde erfolgt vorzugsweise durch Vermischen der Feinstteilchenkomponente mit dem wasserabsorbierenden Polymergebilde in dem Fachmann bekannten Mischvorrichtungen. Nach oder während des Mischens der Feinstteilchenkomponente mit dem wasserabsorbierenden Polymergebilde wird anschließend zumindest ein Teil der Feinstteilchen auf der Oberfläche der wasserabsorbierenden Polymergebilde immobilisiert, wobei das Immobilisieren vorzugsweise durch Erhitzen erfolgt; Dabei ist es insbesondere bevorzugt, dass die Immobilisierung durch Erhitzen auf eine Temperatur erfolgt, die maximal 10 %, besonders bevorzugt maximal 7,5 % und am meisten bevorzugt maximal 5 % über der Erweichungstemperatur eines Bestandteils der Feinstteilchenkomponente, vorzugsweise über der Erweichungstemperatur des Binders, erfolgt. Besonders bevorzugt erfolgt das Erhitzen auf eine Temperatur in einem Bereich von 30 bis 200°C, darüber hinaus bevorzugt von 50 bis 160°C, und am meisten bevorzugt von 100 bis 140°C.

Grundsätzlich sind dabei mindestens vier Vorgehensweisen denkbar:

- Gemäß der Variante $V_A$ wird zunächst die Mischung aus Feinstteilchenkomponente und wasserabsorbierendem Polymergebilde hergestellt und diese anschließend Zwecks Immobilisierung der Feinstteilchen auf die vorstehend genannte Temperatur erhitzt.

- Gemäß der Variante $V_B$ werden zunächst vor dem Vermischen mit der Feinstteilchenkomponente die wasserabsorbierenden Polymergebilde auf die vorstehend beschriebene Temperatur erhitzt und anschließend diese vorgewärmten wasserabsorbierenden Polymergebilde mit der nicht-vorgewärmten Feinstteilchenkomponente vermischt.

- Gemäß der Variante $V_C$ werden zunächst vor dem Vermischen mit der Feinstteilchenkomponente die wasserabsorbierenden Polymergebilde und die Feinstteilchenkomponente getrennt jeweils auf die vorstehend beschriebene Temperatur erwärmt und anschließend die vorgewärmten wasserabsorbierenden Polymergebilde mit der ebenfalls vorgewärmten Feinstteilchenkomponente vermischt. Gemäß einer besonderen Ausgestaltung dieser Variante $V_C$ ist es bevorzugt, die Feinstteilchenkomponente nach dem Erwärmen und vor dem Vermischen mit den vorgewärmten wasserabsorbierenden Polymergebilden zunächst abzukühlen, vorzugsweise auf eine Temperatur in einem Bereich von 10 bis 100°C, besonders bevorzugt 15 bis 75°C und am meisten bevorzugt 20 bis 60°C, danach gegebenenfalls zu zerkleinern, beispielsweise mittels eines Mörsers, und dann die abgekühlte und gegebenenfalls zerkleinerte

Feinstteilchenkomponente mit den vorgewärmten wasserabsorbierenden Polymergebilden zu vermischen.

- Gemäß der Variante $V_D$ wird zunächst vor dem Vermischen mit der Feinstteilchenkomponente die Feinstteilchenkomponente auf die vorstehend beschriebene Temperatur erwärmt und anschließend die vorgewärmte Feinstteilchenkomponente mit den nicht-vorgewärmten wasserabsorbierenden Polymergebilde vermischt. Gemäß einer besonderen Ausgestaltung dieser Variante $V_D$ ist es bevorzugt, die Feinstteilchenkomponente nach dem Erwärmen und vor dem Vermischen mit den nicht-vorgewärmten wasserabsorbierenden Polymergebilden zunächst abzukühlen, vorzugsweise auf eine Temperatur in einem Bereich von 10 bis 100°C, besonders bevorzugt 15 bis 75°C und am meisten bevorzugt 20 bis 60°C, danach gegebenenfalls zu zerkleinern, beispielsweise mittels eines Mörsers, und dann die abgekühlte und gegebenenfalls zerkleinerte Feinstteilchenkomponente mit den nicht-vorgewärmten wasserabsorbierenden Polymergebilden zu vermischen.

Die Formulierung "nicht-vorgewärmt" bedeutet dabei vorzugsweise, dass die Temperatur der jeweiligen Komponente weniger als 100°C, besonders bevorzugt weniger als 80°C und am meisten bevorzugt weniger als 40°C beträgt.

Die Dauer des Erhitzens liegt, je nach Mischgeschwindigkeit und verwendeter Mischvorrichtung vorzugsweise in einem Bereich von 10 Sekunden bis 60 Minuten, besonders bevorzugt in einem Bereich von 30 Sekunden bis 30 Minuten.

Weiterhin kann es vorteilhaft sein, wenn sich an den Verfahrensschritt iv) noch ein weiterer Verfahrensschritt v) anschließt, in dem die wasserabsorbierenden Polymergebilde, auf deren Oberflächen die Feinstteilchen immobilisiert sind, noch für eine Zeitraum in einem Bereich von 10 Minuten bis 5 Stunden, besonders bevorzugt von 30 Minuten bis 3 Stunden gemischt wird, um eine möglichst homogene Verteilung der Feinstteilchen und der absorbierenden Polymergebilde zu ermöglichen, wobei hierzu dem Fachmann bekannte Mischvorrichtungen eingesetzt werden können. In diesem weiteren Verfahrensschritt kann die Superabsorberzusammensetzung mit der Temperatur, die sie nach dem Immobilisieren im Verfahrensschritt iv) aufweist, in den Mischer eingebracht werden, wobei die wasserabsorbierenden Polymergebilde, auf deren Oberflächen die Feinstteilchen immobilisiert sind, dann im Verlaufe des Mischens vorzugsweise stetig auf eine niedrigere Temperatur, vorzugsweise auf Raumtemperatur, abgekühlt werden kann.

Einen weiteren Beitrag zur Lösung der eingangs beschriebenen Aufgaben liefern die durch das erfindungsgemäße Verfahren erhältlichen, oberflächenbehandelten wasserabsorbierenden Polymergebilde.

Dabei ist es bevorzugt, dass die eingangs beschriebenen wasserabsorbierenden Polymergebilde die gleichen Eigenschaften aufweisen wie die durch das erfindungsgemäßen Verfahren erhältlichen oberflächenbehandelten wasserabsorbierenden Polymergebilde. Es ist auch erfindungsgemäß bevorzugt, dass diejenigen Werte, die im Zusammenhang mit dem erfindungsgemäßen Verfahren und den erfindungsgemäßen Polymergebilden als Untergrenzen von erfindungsgemäßen Merkmalen ohne Obergrenzen angegeben wurden, das 20-fache, vorzugsweise das 10-fache und besonders bevorzugt das 5-fache des am meisten bevorzugten Wertes der Untergrenze besitzen.

Einen weiteren Beitrag zur Lösung der eingangs beschriebenen Aufgaben liefert ein Verbund, beinhaltend die erfindungsgemäßen oberflächenbehandelten wasserabsorbierenden Polymergebilde bzw. die durch das erfindungsgemäße Verfahren erhältlichen oberflächenbehandelten wasserabsorbierenden Polymergebilde und ein Substrat. Es ist dabei bevorzugt, dass die erfindungsgemäßen Polymergebilde und das Substrat miteinander fest verbunden sind. Als Substrate sind Folien aus Polymeren, wie beispielsweise aus Polyethylen, Polypropylen oder Polyamid, Metalle, Vliese, Fluff, Tissues, Gewebe, natürliche oder synthetische Fasern, oder andere Schäume bevorzugt. Weiterhin ist es erfindungsgemäß bevorzugt, dass der Verbund mindestens einen Bereich umfasst, welcher das erfindungsgemäße wasserabsorbierende Polymergebilde in einer Menge im Bereich von etwa 15 bis 100 Gew.%, vorzugsweise etwa 30 bis 100 Gew.-%, besonders bevorzugt von etwa 50 bis 99,99 Gew.-%, ferner bevorzugt von etwa 60 bis 99,99 Gew.-% und darüber hinaus bevorzugt von etwa 70 bis 99 Gew.-%, jeweils bezogen auf das Gesamtgewicht des betreffenden Bereichs des Verbundes, beinhaltet, wobei dieser Bereich vorzugsweise eine Größe von mindestens 0,01 $cm^3$, vorzugsweise mindestens 0,1 $cm^3$ und am meisten bevorzugt mindestens 0,5 $cm^3$ aufweist.

In einer besonders bevorzugten Ausführungsform des erfindungsgemäßen Verbundes handelt es sich um einen flächenförmigen Verbund, wie er in der WO-A-02/056812 als "absorbent material" beschrieben ist. Der Offenbarungsgehalt der WO-A-02/056812, insbesondere hinsichtlich des genauen Aufbaus des Verbundes, des Flächengewichtes seiner Bestandteile sowie seiner Dicke wird hiermit als Referenz eingeführt und stellt einen Teil der Offenbarung der vorliegenden Erfindung dar.

Einen weiteren Beitrag zur Lösung der Eingangs genannten Aufgaben liefert ein Verfahren zur Herstellung eines Verbundes, wobei die erfindungsgemäßen wasserabsorbierenden Polymergebilde bzw. die durch das erfindungsgemäße Verfahren erhältlichen oberflächenbehandelten wasserabsorbierenden Polymergebilde und ein Substrat und gegebenenfalls ein Zusatzstoff miteinander in Kontakt gebracht werden. Als Substrate werden vorzugsweise diejenigen Substrate eingesetzt, die bereits vorstehend im Zusammenhang mit dem erfindungsgemäßen Verbund genannt wurden.

Gemäß einer besonderen Ausgestaltung des erfindungsgemäßen Verfahrens zur Herstellung eines Verbundes umfasst dieses Verfahren die folgenden Verfahrensschritte:

I) Bereitstellen eines Substrates;

II) Bereitstellen eines zumindest oberflächenvernetzten, wasserabsorbierenden Polymergebildes, dessen Oberfläche mit einer Verbindung umfassend ein Polykation und mindestens ein Anion in Kontakt gebracht worden ist;

III) Bereitstellen einer eine Vielzahl von Feinstteilchen beinhaltenden Feinstteilchenkomponente;

IV) In Kontakt bringen des Substrates mit dem zumindest oberflächenvemetzten, wasserabsorbierenden Polymergebilde;

V) in Kontakt bringen des zumindest oberflächenvernetzten, wasserabsorbierenden Polymergebildes mit der Feinstteilchenkomponente;

VI) Immobilisieren mindestens eines Teils der Feinstteilchen auf der Oberfläche des Polymergebildes.

Als Feinstteilchenkomponente und als Verbindung umfassend ein Polykation und mindestens ein Anion sind dabei diejenige Feinstteilchenkomponenten und Verbindungen bevorzugt, die bereits vorstehend im Zusammenhang mit den erfindungsgemäßen wasserabsorbierenden Polymergebilden bzw. im Zusammenhang mit dem erfindungsgemäßen Verfahren zur Behandlung der Oberfläche wasserabsorbierender Polymergebilde als bevorzugte Verbindungen bzw. Feinstteilchenkomponente beschrieben worden sind.

Gemäß einer Variante dieser besonderen Ausgestaltung des erfindungsgemäßen Verfahrens zur Herstellung eines Verbundes werden zunächst das Substrat und das oberflächennachvernetzte, mit einer Verbindung umfassend ein Polykation und mindestens ein Anion in Kontakt gebrachte Polymergebilde miteinander in Kontakt gebracht, vorzugsweise dadurch, dass zunächst das Substrat vorgelegt und anschließend das oberflächennachvernetzte Polymergebilde entweder gleichmäßig oder aber auf bestimmte Areale der Substratoberfläche aufgebracht, vorzugsweise gestreut wird. Anschließend werden dann die auf der Substratoberfläche befindlichen wasserabsorbierenden Polymergebilde mit der Feinstteilchenkomponente in Kontakt gebracht, beispielsweise dadurch, dass die Feinstteilchenkomponente auf die auf der Substratoberflächen befindlichen oberflächennachvernetzten Polymergebilde gestreut werden. Schließlich erfolgt die Immobilisierung der Feinstteilchenkomponente auf der Oberfläche des Polymergebildes, wobei diese Immobilisierung vorzugsweise durch das vorstehend im Zusammenhang mit dem erfindungsgemäßen Verfahren zur Behandlung der Oberfläche wasserabsorbierender Polymergebilde beschriebene Erhitzen erfolgt. Bei dieser Variante der besonderen Ausgestaltung des erfindungsgemäßen Verfahrens zur Herstellung eines Verbundes erfolgt mithin der Verfahrensschritt V) nach dem Verfahrensschritt IV).

Gemäß einer anderen Variante dieser besonderen Ausgestaltung des erfindungsgemäßen Verfahrens zur Herstellung eines Verbundes wird zunächst das Substrat vorgelegt. Anschließend wird das oberflächennachvernetzte Polymergebilde mit dem Substrat in Kontakt gebracht, vorzugsweise dadurch, dass zunächst das Substrat vorgelegt und anschließend das oberflächennachvernetzte Polymergebilde entweder gleichmäßig oder aber auf bestimmte Areale der Substratoberfläche auf gebracht, vorzugsweise gestreut wird. Noch bevor das Polymergebilde mit der Substratoberfläche in Kontakt gebracht wird, werden die wasserabsorbierenden Polymergebilde mit der Feinstteilchenkomponente in Kontakt gebracht, beispielsweise dadurch, dass die Feinstteilchenkomponente mit dem oberflächennachvernetzten Polymergebilde, bevor es auf die Substratoberfläche gestreut wird, vermischt wird. Nach dem die Polymergebilde mit dem Substrat in Kontakt gebracht wurden, erfolgt dann die Immobilisierung der Feinstteilchenkomponente auf der Oberfläche des Polymergebildes. Bei dieser Variante der besonderen Ausgestaltung des erfindungsgemäßen Verfahrens zur Herstellung eines Verbundes erfolgt mithin der Verfahrensschritt V) vor dem Verfahrensschritt IV).

Einen Beitrag zur Lösung der Eingangs genannten Aufgaben liefert auch ein Verbund erhältlich nach dem vorstehend beschriebenen Verfahren, wobei dieser Verbund vorzugsweise die gleichen Eigenschaften aufweist wie der vorstehend beschriebene, erfindungsgemäße Verbund.

Einen weiteren Beitrag zur Lösung der Eingangs genannten Aufgaben liefern chemische Produkte beinhaltend die erfindungsgemäßen wasserabsorbierenden Polymergebilde oder einen erfindungsgemäßen Verbund. Bevorzugte chemische Produkte sind insbesondere Schäume, Formkörper, Fasern, Folien, Filme, Kabel, Dichtungsmaterialien, flüssigkeitsaufnehmenden Hygieneartikel, insbesondere Windeln und Damenbinden, Träger für pflanzen- oder pilzwachstumsregulierende Mittel oder Pflanzenschutzwirkstoffe, Zusätze für Baustoffe, Verpackungsmaterialien oder Bodenzusätze.

Auch die Verwendung der erfindungsgemäßen wasserabsorbierenden Polymergebilde oder des erfindungsgemäßen Verbundes in chemischen Produkten, vorzugsweise in den vorstehend genannten chemischen Produkten, insbesondere in Hygieneartikeln wie Windeln oder Damenbinden, sowie die Verwendung der Superabsorberpartikel als Träger für pflanzen- oder pilzwachstumsregulierende Mittel oder Pflanzenschutzwirkstoffe liefern einen Beitrag zur Lösung der eingangs genannten Aufgaben. Bei der Verwendung als Träger für pflanzen- oder pilzwachstumsregulierende

Mittel oder Pflanzenschutzwirkstoffe ist es bevorzugt, dass die pflanzen- oder pilzwachstumsregulierende Mittel oder Pflanzenschutzwirkstoffe über einen durch den Träger kontrollierten Zeitraum abgegeben werden können.

Einen weiteren Beitrag zur Lösung der Eingangs genannten Aufgabe liefert die Verwendung einer Verbindung umfassend mindestens ein Anion und ein Polykation der Struktur I

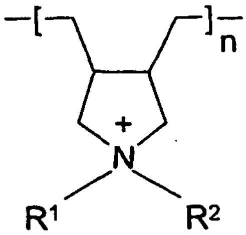

Struktur I

in der

$R^1$ und $R^2$ gleich oder verschieden sein können und ein Kohlenwasserstoffrest mit 1 bis 10 Kohlenstoffatomen, vorzugsweise 1 bis 7 Kohlenstoffatomen, besonders bevorzugt 1 bis 4 Kohlenstoffatomen, darüber hinaus bevorzugt 1 bis 2 Kohlenstoffatomen und am meisten bevorzugt eine Methylgruppe darstellen und

n einen Wert von mindestens 25, vorzugsweise mindestens 31, besonders bevorzugt mindestens 100, noch mehr bevorzugt mindestens 1.000, noch mehr bevorzugt mindestens 2.000, darüber hinaus bevorzugt mindestens 2.500, noch mehr bevorzugt mindestens 3.000 und am meisten bevorzugt mindestens 5.000 hat,

- zur Behandlung der Oberfläche wasserabsorbierender Polymergebilde, und/oder
- zur Verbesserung der Permeabilität von Verbunden umfassend diese Polymergebilde und ein Substrat, wobei die Polymergebilde in dem Verbund in einer Menge von mindestens 50 Gew.-%, vorzugsweise mindestens 70 Gew.-% und am meisten bevorzugt mindestens 90 Gew.-%, bezogen auf das Gesamtgewicht aus Polymergebilde und Substrat, in dem Verbund enthalten sind.

Die Erfindung wird nun anhand von Testmethoden und nicht limitierenden Beispielen näher erläutert.

## TESTMETHODEN

BESTIMMUNG DER PERMEABILITÄT (SFC-TEST, GEMÄß WO-A-95/22356)

In einen Zylinder mit Siebboden werden ca. 0,9 g Superabsorbermaterial eingewogen und sorgfältig auf der Siebfläche verteilt. Das Superabsorbermaterial lässt man in JAYCO synthetischen Urin (Zusammensetzung : 2,0 g Kaliumchlorid; 2,0 g Natriumsulfat; 0,85 g Ammoniumdihydrogenphosphat; 0,15 g Ammoniumhydrogenphosphat ; 0,19 g Calciumchlorid ; 0,23 g Magnesiumchlorid als wasserfreie Salze in1 1 destilliertem Wasser gelöst) 1 Stunde lang gegen einen Druck von 20g/$cm^2$ quellen. Nach Erfassung der Quellhöhe des Superabsorbers lässt man bei konstantem hydrostatischem Druck 0,118 M NaCl-Lösung aus einem nivellierten Vorratsgefäß durch die gequollene Gelschicht laufen. Die gequollene Gelschicht ist während der Messung mit einem speziellen Siebzylinder abgedeckt, der eine gleichmäßige Verteilung der 0,118 M NaCl-Lösung oberhalb des Gels und konstante Bedingungen (Messtemperatur 20-25°C) während der Messung bezüglich der Gelbettbeschaffenheit gewährleistet. Der auf den gequollenen Superabsorber wirkende Druck ist weiterhin 20 g/$cm^2$. Mit Hilfe eines Computers und einer Waage wird die Flüssigkeitsmenge, welche die Gelschicht als Funktion der Zeit passiert, in Intervallen von 20 Sekunden innerhalb einer Zeitperiode von 10 Minuten erfasst. Die Fliessrate in g/s durch die gequollene Gelschicht wird mittels Regressionsanalyse mit Extrapolation der Steigung und Ermittlung des Mittelpunkts auf den Zeitpunkt t=0 der Fliessmenge innerhalb der Minuten 2-10 ermittelt. Der SFC-Wert (K) berechnet sich wie folgt :

$$K = \frac{F_S(t=0) \times L_0}{r \times A \times \Delta P} = \frac{F_S(t=0) \times L_0}{139506}$$

wobei:

| | |
|---|---|
| $F_s(t=0)$ | die Fliessrate in g/s, |
| $L_0$ | die Dicke der Gelschicht in cm, |
| r | die Dichte der NaCl-Lösung (1,003 g/cm$^3$), |
| A | die Fläche der Oberseite der Gelschicht im Messzylinder (28,27 cm$^2$), |
| $\Delta P$ | der hydrostatische Druck, der auf der Gelschicht lastet (4920 dyne/cm$^2$), und |
| K | der SFC-Wert ist [cm$^3$·s-g$^{-1}$] |

BESTIMMUNG DER WICKING-INDEX

Die Bestimmung des Wicking-Index erfolgte gemäß dem auf der Seite 6, Zeile 36 bis Seite 7, Zeile 26 der EP-A-0 532 002 beschriebenen Testverfahren, wobei die Partikel zuvor auf eine Partikelgröße in einem Bereich von 150 bis 850 µm abgesiebt wurden.

BEISPIELE

1. HERSTELLUNG VON SAP-PARTIKELN (NEUTRALISATIONSGRAD 50 MOL-%)

Eine Monomerlösung bestehend aus 300 g Acrylsäure, 166,52 g 50%ige Natronlauge, 497,12 g entionisiertes Wasser, 1,176 g Monoallylpolyethylenglykol-450-monoacrylsäureester, 0,988 g Polyethylenglykol-300-diacrylat und 6,13 g Polyethylenglykol-750-monomethacrylsaureestermethylether wird durch Spülen mit Stickstoff vom gelösten Sauerstoff befreit und auf die Starttemperatur von 4°C abgekühlt. Nach Erreichen der Starttemperatur wurde die Initiatorlösung (0,3 g Natriumperoxydisulfat in 9,7 g $H_2O$ 0,07 g 35,5%ige Wasserstoffperoxid-Lösung in 1,93 g $H_20$ und 0,015 g Ascorbinsäure in 4,99 g $H_20$) zugesetzt. Nachdem die Endtemperatur von ca. 100°C erreicht war, wurde das entstandene Gel zerkleinert und bei 150°C 120 Minuten lang getrocknet. Das getrocknete Polymerisat wurde grob zerstoßen, gemahlen und auf ein Pulver mit einer Partikelgröße von 150 bis 850 µm gesiebt (=Pulver A).

2. HERSTELLUNG VON SAP-PARTIKELN (NEUTRALISATIONSGRAD 70 MOL-%)

Eine Monomerlösung bestehend aus 300 g Acrylsäure, 233,12 g 50%ige Natronlauge, 429,42 g entionisiertes Wasser, 1,961 g Monoallylpolyethylenglykol-450-monoacrylsäureester, 2,372 g Polyethylenglykol-300-diacrylat und 6,13 g Polyethylenglykol-750-monomethaerylsäurcestermethylether wird durch Spülen mit Stickstoff vom gelösten Sauerstoff befreit und auf die Starttemperatur von 4°C abgekühlt. Nach Erreichen der Starttemperatur wurde die Initiatorlösung (0,3 g Natriumperoxydisulfat in 9,7 g $H_2O$ 0,07 g 35,5%ige Wasserstoffperoxid-Lösung in 1,93 g $H_20$ und 0,015 g Ascorbinsäure in 4,99 g $H_20$) zugesetzt. Nachdem die Endtemperatur von ca. 100°C erreicht war, wurde das entstandene Gel zerkleinert und bei 150°C 120 Minuten lang getrocknet. Das getrocknete Polymerisat wurde grob zerstoßen, gemahlen und auf ein Pulver mit einer Partikelgröße von 150 bis 850 µm gesiebt (=Pulver B).

Beispiele 1 und 2 (SAP-Polymer mit 70 Mol-% Neutralisationsgrad)

Das Pulver B wurde mit einer Lösung beinhaltend die in der nachfolgenden Tabelle 1 angegebenen Mengen an Wasser, Ethylencarbonat als Nachvernetzer und Polydiallyldimethylammoniumchlorid (MW=450.000 g/mol) unter kräftigem Rühren vermischt und anschließend für die in der Tabelle 1 angegebene Dauer auf die ebenfalls in der Tabelle 1 angegebenen Temperaturen erhitzt (die angegebenen Mengen an Wasser, Ethylencarbonat und Polydiallyldimethylammoniumchlorid sind jeweils die Mengen in Gramm, bezogen auf 100 g des Pulvers B):

Tabelle 1

| Beispiel | Wasser | Ethylencarbonat | Polydiallyldimethyl-Ammoniumchlorid (als 30%ige Lösung in $H_2O$) | Zeit [Minuten] | Temperatur [°C] |
|---|---|---|---|---|---|
| 1 (nicht erfindungsgemäß) | 2,5 | 1 | 0 | 75 | 190 |
| 2 (erfindungsgemäß) | 0 | 1 | 3 | 75 | 190 |

Die Absorptionseigenschaften der in den Beispielen 1 und 2 erhaltenen Polymere sind in der folgenden Tabelle 2 aufgerührt:

Tabelle 2

| Polymer aus Beispiel | Wicking-Index [cm] | SFC-Wert [$cm^3 \cdot s\text{-}g^{-1}$] | Retention [g/g] |
|---|---|---|---|
| 1 | 5,5 | 127 | 22,5 |
| 2 | 7,5 | 199 | 22,7 |

Beispiele 3 und 4 (SAP-Polymer mit 50 Mol% Neutralisationsgrad)

Das Pulver A wurde mit einer Lösung beinhaltend die in der nachfolgenden Tabelle 3 angegebenen Mengen an Wasser, Ethylencarbonat als Nachvernetzer und Polydiallyldimethylammoniumchlorid (MW=450.000 g/mol) unter kräftigem Rühren vermischt und anschließend für die in der Tabelle 3 angegebene Dauer auf die ebenfalls in der Tabelle 3 angegebenen Temperaturen erhitzt (die angegebenen Mengen an Wasser, Ethylencarbonat und Polydiallyldimethylammoniumchlorid sind jeweils die Mengen in Gramm, bezogen auf 100 g des Pulvers A):

Tabelle 3

| Beispiel | Wasser | Ethylencarbonat | Polydiallyldimethyl-Ammoniumchlorid (als 30%ige Lösung in $H_2O$) | Zeit [Minuten] | Temperatur [°C] |
|---|---|---|---|---|---|
| 3 (nicht erfindungsgemäß) | 2,5 | 1 | 0 | 30 | 180 |
| 4 (erfindungsgemäß) | 0 | 1 | 3 | 30 | 180 |

Die Absorptionseigenschaften der in den Beispielen 3 und 4 erhaltenen Polymere sind in der folgenden Tabelle 4 aufgeführt:

Tabelle 4

| Polymer aus Beispiel | Wicking-Index [cm] | SFC-Wert [$cm^3 \cdot s \cdot g^{-1}$] | AAP [g/g] | Retention [g/g] |
|---|---|---|---|---|
| 3 | 7,0 | 151 | 19,5 | 21,9 |
| 4 | 10,5 | 249 | 20,3 | 20,9 |

Beispiel 5

100 g des im Beispiel 4 erhaltenen Polymers wurden im Trockenschrank auf 130°C vorgewärmt. Eine Mischung aus 10 g $Al_2(SO_4)_3$x14 $H_2O$, welches in einer Zentrifugalmühle gemahlen und auf eine Partikelgröße in einem Bereich von 300 bis 400 μm abgesiebt wurde, und 1,5 g Polyethylenglykol 10.000 (Polyethylenglykol mit einem Molekulargewicht von 10.000 g/mol), welches ebenfalls in einer Zentrifugalmühle gemahlen und auf eine Partikelgröße von weniger als 300 μm abgesiebt wurde), wurde hergestellt. 1,15 g der Mischung aus $Al_2(SO_4)_3 \times 14\ H_2O$ und Polyethylenglykol 10.000 wurden in einem Krups-Mixer unter Rühren mit dem vorgewärmten wasserabsorbierenden Polymergebilde vermischt.

**Patentansprüche**

1. Wasserabsorbierendes Polymergebilde, dessen Oberfläche mit einer Verbindung umfassend ein Polykation und mindestens ein Anion in Kontakt gebracht worden ist, wobei die Verbindung umfassend ein Polykation und mindestens ein Anion ein Gewichtsmittel des Molekulargewichtes von mehr als 3,000 g/mol aufweist,
wobei das Polykation die Struktur I

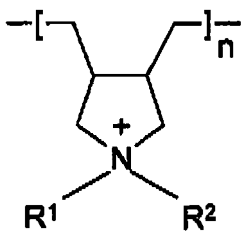

Struktur I

aufweist, in der
$R^1$ und $R^2$ gleich oder verschieden sein können und ein Kohlenwasserstoffrest mit 1 bis 10 Kohlenstoffatomen darstellen und
n einen Wert von mindestens 25 hat,
und wobei das wasserabsorbierende Polymergebilde erhältlich ist durch ein Verfahren umfassend die Verfahrensschritte:

i) Bereitstellen eines zumindest teilweise getrockneten, unbehandelten, wasserabsorbierenden Polymergebildes,
ii) in Kontakt bringen des zumindest teilweise getrockneten, unbehandelten wasserabsorbierenden Polymergebilde mit der mindestens ein Anionen und ein Polykation umfassenden Verbindung,
iii) Nachvernetzung des wasserabsorbierenden Polymergebildes,

wobei der Verfahrensschritt iii) vor, während oder nach dem Verfahrensschritt ii) durchgeführt werden kann.

2. Wasserabsorbierendes Polymergebilde nach Anspruch 1, aufweisend einen Innenbereich und einen den Innenbereich umgebenden Aussenbereich, wobei der Aussenbereich einen höheren Vernetzungsgrad aufweist als der Innenbereich.

3. Polymergebilde nach Anspruch 1, wobei $R^1$ und $R^2$ eine Methylgruppe darstellen.

4. Polymergebilde nach Anspruch 1 oder 3, wobei n einen Wert von mindestens 1.000 hat.

5. Polymergebilde nach Anspruch 1 oder 3, wobei n einen Wert von mindestens 2.000 hat.

6. Polymergebilde nach Anspruch 1 oder 3, wobei n einen Wert von mindestens 3.000 hat.

7. Polymergebilde nach einem der vorhergehenden Ansprüche, wobei das Anion ein Chlorid-Ion ist.

8. Polymergebilde nach einem der vorhergehenden Ansprüche, wobei das Polymergebilde mindestens eine der folgenden Eigenschaften aufweist:

(β1) kein Verbacken nach drei Stunden;
(β2) bei einem CRC-Wert von weniger als 26 g/g einen SFC-Wert (von mindestens $80 \times 10^{-7}$ $cm^3$ s $g^{-1}$;
(β3) bei einem CRC-Wert im Bereich von 26 bis < 27 g/g einen SFC-Wert von mindestens $70 \times 10^{-7}$ $cm^3$ s $g^{-1}$
(β4) bei einem CRC-Wert im Bereich von 27 bis < 28 g/g einen SFC-Wert von mindestens $60 \times 10^{-7}$ $cm^3$ s $g^{-1}$ ;
- (β5) bei einem CRC-Wert im Bereich von 28 bis < 29 g/g einen SFC-Wert von mindestens $45 \times 10^{-7}$$cm^3$ s $g^{-1}$ ;

(β6) bei einem CRC-Wert im Bereich von 29 bist 30 g/g einen SFC-Wert von mindestens $30 \times 10^{-7}$ $cm^3$ $s$ $g^{-1}$;
(β7) bei einem CRC-Wert im Bereich von 30 bis < 31 g/g einen SFC-Wert von mindestens $20 \times 10^{-7} cm^3$ $s$ $g^{-1}$;
(β8) bei einem CRC-Wert von mindestens 31 g/g einen SFC-Wert von mindestens $10 \times 10^{-7} cm^3$ $s$ $g^{-1}$;
(β9) einen Wicking-Index nach einer Minute von mindestens 7,5 cm;
(β10) einen Neutralisationsgrad von höchstens 75 Mol-%.

**9.** Ein Verfahren zur Behandlung der Oberflächen wasserabsorbierender Polymergebilde, umfassend die Verfahrensschritte:

i) Bereitstellen eines zumindest teilweise getrockneten, unbehandelten, wasserabsorbierenden Polymergebildes,
ii) in Kontakt bringen des zumindest teilweise getrockneten, unbehandelten wasserabsorbierenden Polymergebilde mit einer mindestens ein Anionen und ein Polykation umfassenden Verbindung,
iii) Nachvernetzung des wasserabsorbierenden Polyznergebildes,

wobei der Verfahrensschritt iii) vor, während oder nach dem Verfahrensschritt ii) durchgeführt werden kann und wobei das Polykation die Struktur I

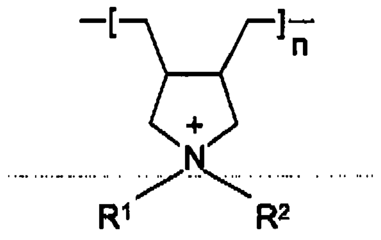

Struktur I

aufweist, in der
$R^1$ und $R^2$ gleich oder verschieden sein können und ein Kohlenwasserstoffrest mit 1 bis 10 Kohlenstoffatomen darstellt und
n einen Wert von mindestens 25 hat.

**10.** Verfahren nach Anspruch 9, wobei das wasserabsorbierende Polymergebilde mit der Verbindung in einer Menge in einem Bereich von 0,001 bis 10 Gew.-%, bezogen auf das Gewicht des wasserabsorbierenden Polymergebildes, in Kontakt gebracht wird.

**11.** Verfahren nach einem der Ansprüche 9 oder 10, wobei die Verbindung in Form einer 10 bis 80 Gew.%igen wässrigen Lösung mit dem wasserabsorbierenden Polymergebilde in Kontakt gebracht wird.

**12.** Verfahren nach einem der Ansprüche 9 bis 11, wobei das wasserabsorbierende Polymergebilden einen Neutralisationsgrad von nicht mehr als 70 Mol% aufweist.

**13.** Wasserabsorbierendes Polymergebilde, erhältlich nach einem Verfahren nach einem der Ansprüche 9 bis 12.

**14.** Verbund, beinhaltend ein wasserabsorbierendes Polymergebilde nach einem der Ansprüche 1 bis 8 oder 13 und ein Substrat.

**15.** Verfahren zur Herstellung eines Verbundes, wobei ein wasserabsorbierendes Polymergebilde nach einem der Ansprüche 1 bis 8 oder 13 und ein Substrat und gegebenenfalls ein Hilfsmittel miteinander in Kontakt gebracht werden.

**16.** Verbund, erhältlich nach einem Verfahren gemäß Anspruch 15.

**17.** Schäume, Formkörper, Fasern, Folien, Filme, Kabel, Dichtungsmaterialien, flüssigkeitsaufnehmende Hygieneartikel, Träger für pflanzen- und pilzwachsturnregulierende Mittel, Verpackungsmaterialien, Bodenzusätze oder Baustoffe, beinhalten das wasserabsorbierende Polymergebilde nach einem der Ansprüche 1 bis 8 oder 13 oder den Verbund nach Anspruch 14 oder 16.

**18.** Verwendung des wasserabsorbierenden Polymergebilde nach einem der Ansprüche 1 bis 8 oder 13 oder des Verbunds nach Anspruch 14 oder 16 in Schäumen, Fonnkörpern, Fasern, Folien, Filmen, Kabeln, Dichtungsmaterialien, flüssigkeitsaufnehmenden Hygieneartikeln, Trägern für pflanzen- und pilzwachstumregulierenden Mitteln, Verpackungsmaterialien, Bodenzusätzen, zur kontrollierten Freisetzung von Wirkstoffen oder in Baustoffen.

**19.** Verwendung einer Verbindung umfassend mindestens ein Anion und ein Polykation der Struktur I

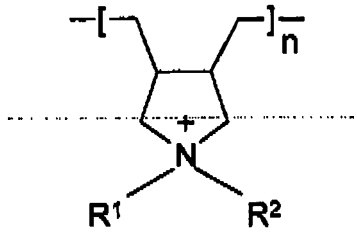

Struktur I

in der
$R^1$ und $R^2$ gleich oder verschieden sein können und ein Kohlenwasserstoffrest mit 1 bis 10 Kohlenstoffatomen darstellen und
n einen Wert von mindestens 25 hat,
zur Behandlung der Oberfläche zumindest teilweise getrockneter, wasserabsorbierender, nachvernetzter Polymergebilde.

## Claims

**1.** Water-absorbent polymer structure, the surface of which has been brought into contact with a compound comprising a polycation and at least one anion, wherein the compound comprising a polycation and at least one anion has a weight average molecular weight of more than 3,000 g/mol,
wherein the polycation has the structure I

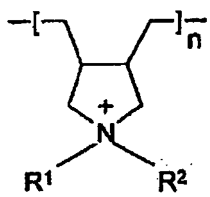

structure I

in which
$R^1$ and $R^2$ can be the same or different and represent a hydrocarbon radical containing 1 to 10 carbon atoms and
n has a value of at least 25,
and wherein the water-absorbent polymer structure is obtainable by a process including the following process steps:

i) providing an at least partially dried, untreated, water-absorbent polymer structure,

ii) bringing the at least partially dried, untreated, water-absorbent polymer structure into contact with the compound comprising at least one anion and a polycation,
iii) post-crosslinking the water-absorbent polymer structure,

wherein process step iii) can be carried out before, during or after process step ii).

**2.** Water-absorbent polymer structure according to claim 1, having an inner region and an outer region surrounding the inner region, wherein the outer region has a higher degree of crosslinking than the inner region.

**3.** Polymer structure according to claim 1, wherein $R^1$ and $R^2$ represent a methyl group.

**4.** Polymer structure according to claim 1 or 3, wherein n has a value of at least 1,000.

**5.** Polymer structure according to claim 1 or 3, wherein n has a value of at least 2,000.

**6.** Polymer structure according to claim 1 or 3, wherein n has a value of at least 3,000.

**7.** Polymer structure according to one of the preceding claims, wherein the anion is a chloride ion.

**8.** Polymer structure according to one of the preceding claims, wherein the polymer structure has at least one of the following properties:

(β1) no caking after three hours;
(β2) at a CRC value of less than 26 g/g, an SFC value of at least 80 x $10^{-7}$ $cm^3$ s $g^{-1}$;
(β3) at a CRC value in the range of from 26 to < 27 g/g, an SFC value of at least 70 x $10^{-7}$ $cm^3$ s $g^{-1}$;
(β4) at a CRC value in the range of from 27 to < 28 g/g, an SFC value of at least 60 x $10^{-7}$ $cm^3$ s $g^{-1}$;
(β5) at a CRC value in the range of from 28 to < 29 g/g, an SFC value of at least 45 x $10^{-7}$ $cm^3$ s $g^{-1}$;
(β6) at a CRC value in the range of from 29 to < 30 g/g, an SFC value of at least 30 x $10^{-7}$ $cm^3$ s $g^{-1}$;
(β7) at a CRC value in the range of from 30 to < 31 g/g, an SFC value of at least 20 x $10^{-7}$ $cm^3$ s $g^{-1}$;
(β8) at a CRC value of at least 31 g/g, an SFC value of at least 10 x $10^{-7}$ $cm^3$ s $g^{-1}$;
(β9) a Wicking index after one minute of at least 7.5 cm;
(β10) a degree of neutralisation of at most 75 mol %.

**9.** A process for the treatment of the surface of water-absorbent polymer entities, including the following process steps:

i) providing an at least partially dried, untreated, water-absorbent polymer structure,
ii) bringing the at least partially dried, untreated, water-absorbent polymer structure into contact with a compound comprising at least one anion and a polycation,
iii) post-crosslinking the water-absorbent polymer structure,

wherein process step iii) can be carried out before, during or after process step ii) and wherein the polycation has the Structure I

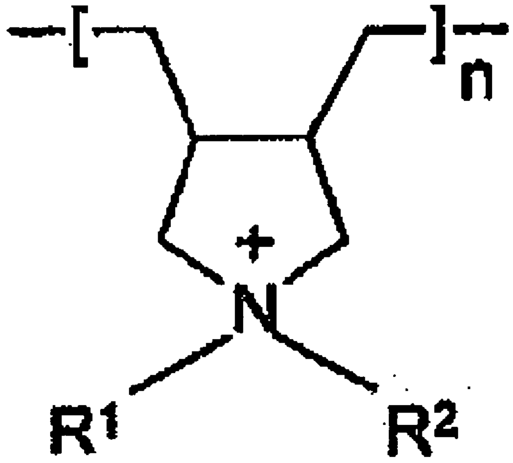

Structure I

in which
$R^1$ and $R^2$ can be the same or different and represent a hydrocarbon radical containing 1 to 10 carbon atoms and n has a value of at least 25.

**10.** Process according to claim 9, wherein the water-absorbent polymer structure is brought into contact with the compound in an amount within a range of from 0.001 to 10 % by weight, based on the weight of the water-absorbent polymer structure.

**11.** Process according to one of claims 9 or 10, wherein the compound, in the form of a 10 to 80 % by weight aqueous solution, is brought into contact with the water-absorbent polymer structure.

**12.** Process according to one of claims 9 to 11, wherein the water-absorbent polymer structure has a degree of neutralisation of not more than 70 mol %.

**13.** Water-absorbent polymer structure, obtainable by a process according to one of claims 9 to 12.

**14.** Composite, containing a water-absorbent polymer structure according to one of claims 1 to 8 or 13 and a substrate.

**15.** Process for preparing a composite, wherein a water-absorbent polymer structure according to one of claims 1 to 8 or 13 and a substrate and optionally an auxiliary are brought into contact with one another.

**16.** Composite, obtainable by a process according to claim 15.

**17.** Foams, moulded bodies, fibres, sheets, films, cables, sealing materials, liquid-absorbing hygiene articles, carriers for plant and fungus growth-regulating agents, packaging materials, soil additives or construction materials, containing the water-absorbent polymer structure according to one of claims 1 to 8 or 13 or the composite according to claim 14 or 16.

**18.** Use of the water-absorbent polymer structure according to one of claims 1 to 8 or 13 or of the composite according to claim 14 or 16 in foams, moulded bodies, fibres, sheets, films, cables, sealing materials, liquid-absorbing hygiene articles, carriers for plant and fungus growth-regulating agents, packaging materials, soil additives, for the controlled release of active ingredients or in construction materials.

**19.** Use of a compound comprising at least one anion and a polycation of Structure I

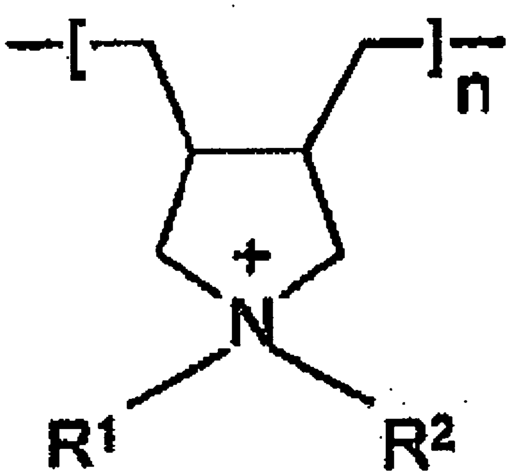

structure I

in which
$R^1$ and $R^2$ can be the same or different and represent a hydrocarbon radical containing 1 to 10 carbon atoms and n has a value of at least 25,
for the treatment of the surface of at least partially dried, water-absorbent, post-crosslinked polymer entities.

**Revendications**

1. Structure polymère absorbant l'eau, dont la surface a été amenée en contact avec un composé comprenant un polycation et au moins un anion, le composé comprenant un polycation et au moins un anion présentant une moyenne pondérale du poids moléculaire de plus de 3.000 g/mol, sachant que le polycation présente la structure I

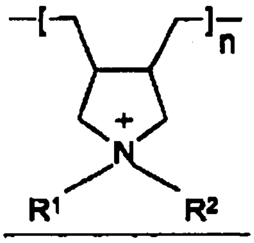

Structure I

dans laquelle$R^1$ et $R^2$ peuvent être identiques ou différents et représentent un radical hydrocarbure avec 1 à 10 atomes de carbone et n a une valeur d'au moins 25, et sachant que la structure polymère absorbant l'eau peut être obtenue par un procédé comprenant les étapes de procédé :

i) Mise à disposition d'une structure polymère au moins partiellement séchée, non traitée, absorbant l'eau,
ii) Mise en contact de la structure polymère au moins partiellement séchée, non traitée, absorbant l'eau avec ledit composé comprenant au moins un anion et un polycation,
iii) Réticulation ultérieure de la structure polymère absorbant l'eau, sachant que l'étape de procédé iii) peut être effectuée avant, pendant ou après l'étape de procédé ii).

2. Structure polymère absorbant l'eau selon la revendication 1, comprenant une zone interne et une zone externe entourant la zone interne, la zone externe présentant un degré de réticulation plus élevé que la zone interne.

3. Structure polymère selon la revendication 1, dans laquelle R1 et R2 représentent un groupe méthyle.

4. Structure polymère selon la revendication 1 ou 3, dans laquelle n a une valeur d'au moins 1.000.

5. Structure polymère selon la revendication 1 ou 3, dans laquelle n a une valeur d'au moins 2.000.

6. Structure polymère selon la revendication 1 ou 3, dans laquelle n a une valeur d'au moins 3.000.

7. Structure polymère selon l'une quelconque des revendications précédentes, dans laquelle l'anion est un ion de chlorure.

8. Structure polymère selon l'une quelconque des revendications précédentes, la structure polymère résentant au moins une des propriétés suivantes :

(β1) pas de prise en masse au bout de trois heures ;
(β2) pour une valeur CRC (capacité de rétention centrifuge) de moins de 26 g/g, une valeur SFC (conductivité en flux salin) d'au moins 80 x $10^{-7}$ $cm^3$ s $g^{-1}$ ;
(β3) pour une valeur CRC dans l'intervalle de 26 à <27 g/g, une valeur SFC d'au moins 70 x $10^{-7}$ $cm^3$ s $g^{-1}$ ;
(β4) pour une valeur CRC dans l'intervalle de 27 à <28 g/g, une valeur SFC d'au moins 60 x $10^{-7}$ $cm^3$ s $g^{-1}$;
(β5) pour une valeur CRC dans l'intervalle de 28 à <29 g/g, une valeur SFC d'au moins 45 x $10^{-7}$ $cm^3$ s $g^{-1}$;
(β6) pour une valeur CRC dans l'intervalle de 29 à <30 g/g, une valeur SFC d'au moins 30 x $10^{-7}$ $cm^3$ s $g^{-1}$ ;
(β7) pour une valeur CRC dans l'intervalle de 30 à <31 g/g, une valeur SFC d'au moins 20 x $10^{-7}$ $cm^3$ s $g^{-1}$;
(β8) pour une valeur CRC d'au moins 31 g/g, une valeur SFC d'au moins 10 x $10^{-7}$ $cm^3$ s $g^{-1}$ ;
(β9) un indice de Wicking au bout d'une minute d'au moins 7,5 cm ;
(β10) un degré de neutralisation d'au plus 75 % en mole.

9. Un procédé de traitement de la surface de structures polymères absorbant l'eau, comprenant les étapes de procédé :

i) Mise à disposition d'une structure polymère au moins partiellement séchée, non traitée, absorbant l'eau,
ii) Mise en contact de la structure polymère au moins partiellement séchée, non traitée, absorbant l'eau avec un composé comprenant au moins un anion et un polycation,
iii) Réticulation ultérieure de la structure polymère absorbant l'eau, sachant que l'étape de procédé iii) peut être effectuée avant, pendant ou après l'étape de procédé ii) et que le polycation présente la structure I

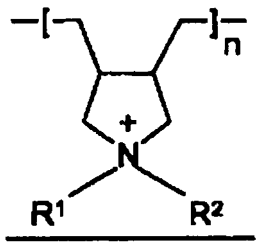

Structure I

dans laquelle$R^1$ et $R^2$ peuvent être identiques ou différents et représentent un radical hydrocarbure avec 1 à 10 atomes de carbone et n a une valeur d'au moins 25.

10. Procédé selon la revendication 9, dans lequel la structure polymère absorbant l'eau est amenée en contact avec le composé en une quantité dans un intervalle de 0,001 à 10 % en poids, rapportée au poids de la structure polymère absorbant l'eau.

11. Procédé selon l'une quelconque des revendications 9 ou 10, dans lequel le composé est amené en contact avec la structure polymère absorbant l'eau sous la forme d'une solution de 10 à 80 % en poids.

12. Procédé selon l'une quelconque des revendications 9 à 11, dans lequel la structure polymère absorbant l'eau

présente un degré de neutralisation de pas plus de 70 % en mole.

**13.** Structure polymère absorbant l'eau pouvant être obtenue d'après un procédé selon l'une quelconque des revendications 9 à 12.

**14.** Composite contenant une structure polymère absorbant l'eau selon l'une quelconque des revendications 1 à 8 ou 13 et un substrat.

**15.** Procédé pour la fabrication d'un composite, pour lequel une structure polymère absorbant l'eau selon l'une quelconque des revendications 1 à 8 ou 13 et un substrat et le cas échéant un agent auxiliaire sont amenés en contact l'un avec l'autre.

**16.** Composite pouvant être obtenu d'après un procédé selon la revendication 15.

**17.** Expansés, corps moulés, fibres, feuilles, films, câbles, matériaux d'étanchéité, articles d'hygiène absorbant les liquides, supports pour produits de régulation de la croissance des plantes et des champignons, matériaux d'emballage, additifs pour sols ou matériaux de construction contenant la structure polymère absorbant l'eau selon l'une quelconque des revendications 1 à 8 ou 13 ou le composite selon la revendication 14 ou 16.

**18.** Utilisation de la structure polymère absorbant l'eau selon l'une quelconque des revendications 1 à 8 ou 13 ou du composite selon la revendication 14 ou 16 dans des expansés, des corps moulés, des fibres, des feuilles, des films, des câbles, des matériaux d'étanchéité, des articles d'hygiène absorbant les liquides, des supports pour produits de régulation de la croissance des plantes et des champignons, des matériaux d'emballage, des additifs pour sols, pour la libération contrôlée de principes actifs ou dans des matériaux de construction.

**19.** Utilisation d'un composé comprenant au moins un anion et un polycation de la structure 1

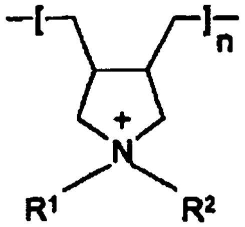

Structure I

dans laquelle$R^1$ et $R^2$ peuvent être identiques ou différents et représentent un radical de hydrocarbure avec 1 à 10 atomes de carbone et n a une valeur d'au moins 25, pour le traitement de la surface de structures polymères au moins partiellement séchées, absorbant l'eau, réticulées ultérieurement.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE OS2612846 A
- DE 4020780 C1
- EP 0248963 A2
- EP 0248973 A2
- DE 19529348 A1
- WO 9934843 A1
- WO 2004037903 A2
- WO 0010619 A1
- US 4286082 A
- DE 2706135
- US 4076663 A
- DE 3503458
- DE 4020780
- DE 4244548
- DE 4323001
- DE 4333056
- DE 4418818
- US 4340706 A
- DE 3713601
- DE 2840010
- WO 9605234 A1
- DE 10334286 A
- WO 02056812 A
- WO 9522356 A
- EP 0532002 A